# EUROPEAN PATENT SPECIFICATION

(11) **EP 2 149 329 B1**
(45) Date of publication and mention of the grant of the patent: **16.09.2015**
(21) Application number: 09009447.5
(22) Date of filing: 21.07.2009
(51) Int. Cl.: A61B 1/01, A61B 1/015, A61B 1/31

(54) **Endoscope insertion aid and endoscope apparatus**
Endoskopische Einführhilfe und endoskopische Vorrichtung
Dispositif d'assistance d'insertion d'endoscope et appareil endoscope

(30) Priority: 28.07.2008 JP 2008193837
(43) Date of publication of application: 03.02.2010
(73) Proprietor: Olympus Medical Systems Corp., Tokyo 151-0072 (JP)
(72) Inventor: Makiyama, Satoshi, Shibuya-ku Tokyo, 151-0072 (JP)
(74) Representative: von Hellfeld, Axel

(56) References cited:
- JP-A- 2003 079 565
- US-A- 5 221 258
- US-A1- 2002 128 559
- US-A1- 2005 222 495
- US-A1- 2007 232 853

## Description

### BACKGROUND OF THE INVENTION

### 1. Field of the Invention

The present invention relates to an endoscope insertion aid and endoscope apparatus suitable for smoothly inserting an insertion portion of an endoscope into an intestinal tract such as the large intestine or small intestine.

### 2. Description of the Related Art

Generally, an endoscope includes an operation portion intended to be gripped by a surgeon and equipped with various operation switches and the like, and an insertion portion inserted into a body cavity. Starting from a distal end, the insertion portion includes a distal end portion, bending portion, and flexible tubular portion, all of which are provided continuously. The flexible tubular portion has flexibility and elongated shape, extending from the operation portion. The bending portion is provided continuously with a distal end of the flexible tubular portion and configured to be bendable in left and right or up and down directions by being operated from the operation portion. The distal end portion has a distal rigid portion configured to be rigid and provided continuously with a distal end of the bending portion. The endoscope is designed to be inserted into the body through the anus, mouth, or nose to allow the surgeon to observe or treat predetermined sites.

Conventionally, when inserting the insertion portion of the endoscope configured as described above into an intestinal tract, the surgeon inserts the insertion portion gradually into the body of a patient by applying a force to the insertion portion from outside the body.

However, the intestinal tract into which the insertion portion of the endoscope is pushed is a soft, internally narrow, long, and intricately winding organ which is not secured to the body. Consequently, when the surgeon pushes the insertion portion of the endoscope into the intestinal tract, the intestinal tract may be moved or squeezed in a forward direction. In that case, when the surgeon relaxes pushing force, the plunged insertion portion may be returned to its original position by reaction force of the intestinal tract, making it difficult to move forward. In particular, the deeper in the intestinal tract, the harder the plunged insertion portion is pushed back. This makes it difficult to insert the insertion portion into a deep part, resulting in longer endoscopy time.

To solve this problem, Japanese Patent Application Laid-Open Publication No. 2007-37649 proposes an endoscope insertion aid which allows the insertion portion of an endoscope to be inserted into an intestinal tract without applying a force from outside the body.

With the endoscope insertion aid proposed in Japanese Patent Application Laid-Open Publication No. 2007-37649, a balloon held by a balloon holding member is detachably mounted on an outer peripheral surface near the distal end portion of the insertion portion of the endoscope. The balloon holding member is coupled with a shaft which, being passed through a channel in the insertion portion, extends from the insertion portion to allow the balloon to move ahead of a distal end portion when the shaft is moved forward and backward on the user's hand side. On the other hand, the balloon is connected with a distal end of a fluid tube to allow the balloon to be supplied with a fluid through the fluid tube, inflated by the fluid, and thereby fixed to the intestinal tract and the like.

With the endoscope insertion aid, when the shaft is drawn to the user's hand side with the inflated balloon secured to the intestinal tract and the like, the insertion portion of the endoscope is moved toward the balloon, allowing the insertion portion to be inserted deep into the intestinal tract.

With the endoscope insertion aid proposed in Japanese Patent Application Laid-Open Publication No. 2007-37649, the shaft is drawn to the user's hand side with the intestinal tract held by the inflated balloon to haul in the intestinal tract with the balloon and thereby move the insertion portion of the endoscope forward. To prevent the hauled intestinal tract from being pushed back by reaction force of the intestinal tract, the surgeon keeps the intestinal tract hauled in by pressing a bending portion or distal end portion against an inner wall of the intestinal tract.

Depending on bend angle of the bending portion, the force of the bending portion or distal end portion holding the intestinal tract is too weak to hold the intestinal tract securely. In such a case, the hauled intestinal tract is pushed back, making it difficult to smoothly insert the insertion portion of the endoscope deep into the intestinal tract.

On the other hand, too large a bend angle of the bending portion may cause pain to the patient, or adverse effects on forward movement of the balloon by subsequent push operation.

In addition to the one proposed in Japanese Patent Application Laid-Open Publication No. 2007-37649, available endoscope insertion aids include an overtube with a balloon. The overtube with a balloon is used for an endoscope for examination of the large or small intestine. The balloon is mounted on a distal end of the overtube which, being configured to be flexible and elongated, allows passage of the insertion portion of the endoscope.

However, such an overtube with a balloon has a slippery interior. Besides, the overtube for the large intestine is configured to cover the entire length of the insertion portion, making it difficult to pull the overtube and insertion portion together when twisting the insertion portion or hauling in the intestinal tract using the balloon or otherwise operate the endoscope together with the overtube.

The present invention has been made in view of the above problems and has an object to provide an endoscope insertion aid and endoscope apparatus which allow the surgeon to draw and haul in an intestinal tract using an insertion portion of an endoscope with the intestinal tract held by a balloon, keep the hauled intestinal tract by fixing a restraining member with a restraining member fixing unit, and thereby straighten the intestinal tract easily and insert the insertion portion deep into the intestinal tract smoothly.

US 5,221,258 relates to an apparatus and method for delivering over-sized or large bore devices through passageways in the body. The apparatus comprises an elongated inflatable balloon having an inner diameter slightly larger than the outer diameter of the device to be introduced into the passageway. A guidewire may be utilized to assist introduction of the balloon into the bodily passageway. A device introduction chamber is attached at the proximal end of the balloon for temporarily housing the device being introduced into the passageway, and the device itself includes a delivery shaft at least partially disposed in the introduction chamber. An inflation device is provided for inflating the balloon with a biologically compatible fluid once the balloon has been inserted into the bodily passageway. The distal portion of the balloon is inserted into the bodily passageway, the balloon is then inflated to expand and straighten the passageway, and the device may then be advanced through the balloon into the passageway. After the device has been advanced within the balloon into the larger, primary passageway, the balloon may be ruptured to free the device from the balloon, allowing to the device to be further advanced through the passageway to a destination such as the heart.

US 2007/0232853 A1 relates to an endoscope insertion assisting device, an endoscope apparatus, a medical treatment device and an endoscope insertion method, wherein a first balloon member having a first hollow portion that allows an insertion portion of the endoscope to pass therethrough, a first transmission member that transmits an advance/retreat action performed by an operator to the first balloon member, a second balloon member having a second hollow portion that allows the insertion portion of the endoscope to pass therethrough, a second transmission member that transmits an advance/retreat action performed by an operator to the second balloon member, and a control portion that controls supply and exhaust of fluid to and from the inside of the first balloon member and the second balloon member to expand or contract the first balloon member and the second balloon member are provided.

US 2002/0128559 A1 refers to a hyperspectral imaging endoscopy apparatus that utilizes spectral technology to acquire, process and exploit gastroscopic data. The apparatus allows for real time anomaly detection and identification. The apparatus includes an endoscope, a spectrometer and a processing unit that perform hyerspectral analysis on spectral data generated from the spectrometer. The endoscope and associated coupling optics are preferably optimized for cylindrical symmetry, thus allowing continuous inspection of gastrointestinal or arterial walls.

### SUMMARY OF THE INVENTION

The present invention provides an endoscope insertion aid including: a tubular member which allows an insertion portion of an endoscope to be passed through and can move forward and backward relatively along an insertion axis of the passed insertion portion; a holding unit which, being provided on an outer peripheral side of the tubular member, can be inflated and deflated with supply and discharge of a fluid; a thread-like member passed through a channel in the insertion portion, from an opening portion on a distal end side of the endoscope to an opening on a proximal end side of the endoscope, the thread-like member having one end connected to the tubular member and the other end extended from the opening; a thread-like member fixing unit which restrains the thread-like member extending from the channel opening provided on the proximal end side of the endoscope and thereby restricts movement of the tubular member toward the proximal end side with respect to the insertion portion along the insertion axis; a restraining member which supplies and discharges the fluid into/from the holding unit, being a tube conduit member being connected at one end to the tubular member, and extending at the other end to the proximal end side along an outer peripheral side of the insertion portion; and a restraining member fixing unit which holds the tubular member at a desired position in an intestinal tract by fixing the other end of the restraining member.

The above and other objects, features and advantages of the invention will become more clearly understood from the following description referring to the accompanying drawings.

### BRIEF DESCRIPTION OF THE DRAWINGS

Fig. 1 is a diagram showing an overall configuration of an endoscope apparatus equipped with an endoscope insertion aid according to a first embodiment of the present invention;
Fig. 2 is a perspective view illustrating a configuration of the endoscope insertion aid mounted on a proximal side of a bending portion of an insertion portion;
Fig. 3 is a perspective view showing a variation of the endoscope insertion aid which differs from Fig. 2 in placement location with respect to the insertion portion;
Fig. 4 is partial sectional view illustrating the configuration of the endoscope insertion aid inserted in the insertion portion shown in Fig. 2;
Fig. 5 is an exploded perspective view illustrating the configuration of the endoscope insertion aid shown in Fig. 2;
Fig. 6 is a perspective view illustrating a configuration of a restraining member fixing unit which restricts the restraining member;
Fig. 7 is a perspective view illustrating a configuration of a fixing unit which fixes a thread-like member;
Fig. 8 is a perspective view illustrating a configuration of a thread-like member restricting unit which restricts the thread-like member;
Fig. 9 is a diagram showing how the thread-like member is restrained;
Fig. 10 is a diagram illustrating how the insertion portion is inserted using the endoscope insertion aid according to the first embodiment, where the insertion portion is inserted through the anus with a balloon tube member fixed;
Fig. 11 is a diagram showing how the insertion portion is inserted from the rectum to the sigmoid colon portion by bending operation and the like from a state shown in Fig. 10;
Fig. 12 is a diagram showing how the sigmoid colon portion is held by inflating a balloon in a state shown in Fig. 11;
Fig. 13 is a diagram showing how an intestinal tract is hauled in and straightened by drawing the insertion portion and fluid tube in a state shown in Fig. 12;
Fig. 14 is a diagram showing how the balloon tube member is locked by holding the fluid tube using a tube fixing member in a state shown in Fig. 13;
Fig. 15 is a diagram showing how the insertion portion is inserted further into the splenic flexure in a deep part of the intestinal tract by releasing the thread-like member from a thread fixing unit in a state shown in Fig. 14;
Fig. 16 is a diagram illustrating how the insertion portion is pulled out using the endoscope insertion aid according to a first embodiment, where a distal side of the insertion portion has reached the cecum in a state shown in Fig. 15;
Fig. 17 is a diagram showing how the insertion portion is drawn by releasing the fluid tube from the tube fixing member in the state shown in Fig. 15;
Fig. 18 is a diagram showing how the insertion portion and fluid tube are drawn and pulled out of the anus by deflating the balloon in a state shown in Fig. 16;
Fig. 19 is an explanatory diagram showing how an improved thread-like member is led out of a second channel entrance port, according to a second embodiment of the present invention;
Fig. 20 is a perspective view illustrating a configuration of an improved rotary-dial thread fixing unit;
Fig. 21 is a perspective view showing how the rotary-dial thread fixing unit shown in Fig. 20 is mounted near the second channel entrance port of the endoscope;
Fig. 22 is a sectional view of a mounting portion of the endoscope and rotary-dial thread fixing unit shown in Fig. 21;
Fig. 23 is an exploded block diagram showing a concrete configuration of the rotary-dial thread fixing unit shown in Fig. 20;
Fig. 24 is a block diagram of the rotary-dial thread fixing unit shown in Fig. 23 as viewed from a mounting direction of the endoscope;
Fig. 25 is a block diagram of the rotary-dial thread fixing unit shown in Fig. 23 as viewed from above;
Fig. 26 is a block diagram of the rotary-dial thread fixing unit shown in Fig. 23 as viewed from below;
Fig. 27 is an exploded perspective view illustrating a configuration of principal part of the rotary-dial thread fixing unit shown in Fig. 20;
Fig. 28 is a sectional view taken along line XXVIII - XXVIII in Fig. 23;
Fig. 29 is a sectional view taken along line XXIX - XXIX in Fig. 28;
Fig. 30 is a sectional view near an elastic portion of the thread-like member used in the present embodiment;
Fig. 31 is an explanatory diagram illustrating the operation of restraining the elastic portion of the thread-like member in a grooved portion of a rotating shaft of the thread fixing unit;
Fig. 32 is an explanatory diagram showing a maximum stroke through which the balloon tube member can move with respect to the insertion portion;
Fig. 33 is an explanatory diagram showing a return of the balloon tube member when the thread-like member is wound by rotating a rotary dial;
Fig. 34 is a partial cutaway, sectional view illustrating a configuration of an endoscope insertion aid equipped with inner balloons instead of the thread-like member, according to a third embodiment of the present invention;
Fig. 35 is a block diagram as viewed along arrow XXXV in Fig. 34;
Fig. 36 is a sectional view taken along line XXXVI - XXXVI in Fig. 34; and
Fig. 37 is an exploded perspective view of the endoscope insertion aid in Fig. 34.

### DETAILED DESCRIPTION OF PREFERRED EMBODIMENTS

Embodiments of the present invention will be described below with reference to the drawings.

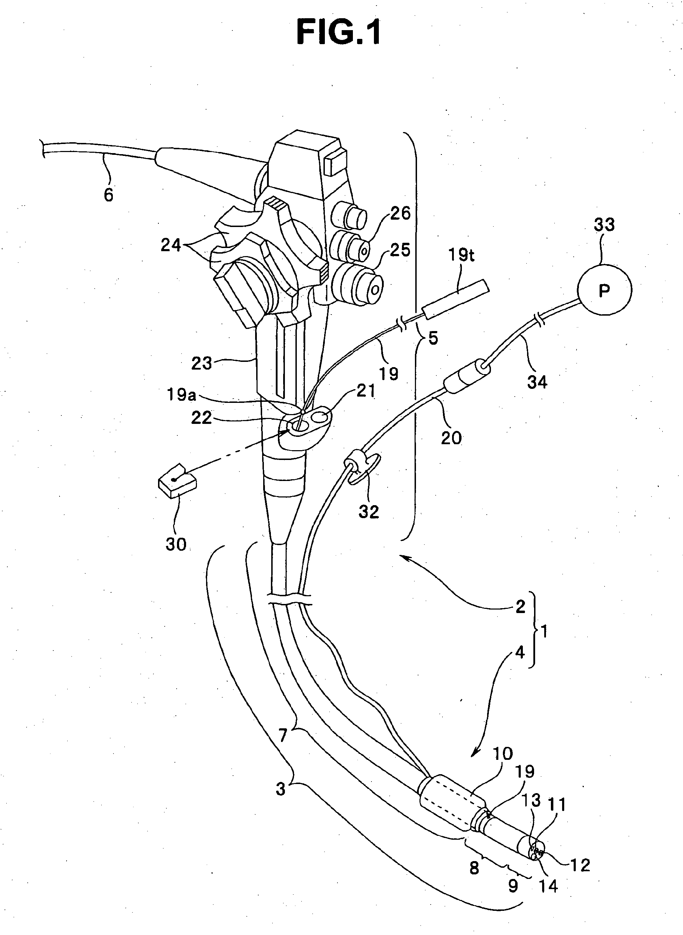

As shown in Fig. 1, an endoscope apparatus 1 according to a first embodiment includes an endoscope 2 used for endoscopy and an endoscope insertion aid 4 which assists insertion of the endoscope 2. An insertion portion 3 of the endoscope 2 is inserted into the body, for example, an intestinal tract. The endoscope insertion aid 4 is detachably mounted on a distal side of the insertion portion 3 of the endoscope 2.

The endoscope 2 includes the insertion portion 3 inserted into the intestinal tract and the like, an operation portion 5 provided at a proximal end of the insertion portion 3, and a universal cable 6 which extends form a lateral portion of the operation portion 5. The universal cable 6 has a connector (not shown) in an end portion. The connector is detachably connected to a light source device or signal processing device.

The insertion portion 3 includes a flexible tubular portion 7 which has flexibility and elongated shape, a bending portion 8 which is bendable and provided at a distal end of the flexible tubular portion 7, and a rigid, distal end portion 9 provided at a distal end of the bending portion 8.

As shown in Fig. 1, a balloon 10 is mounted on an outer peripheral side on the distal side of the flexible tubular portion 7 of the insertion portion 3 to provide a holding unit for the endoscope insertion aid 4.

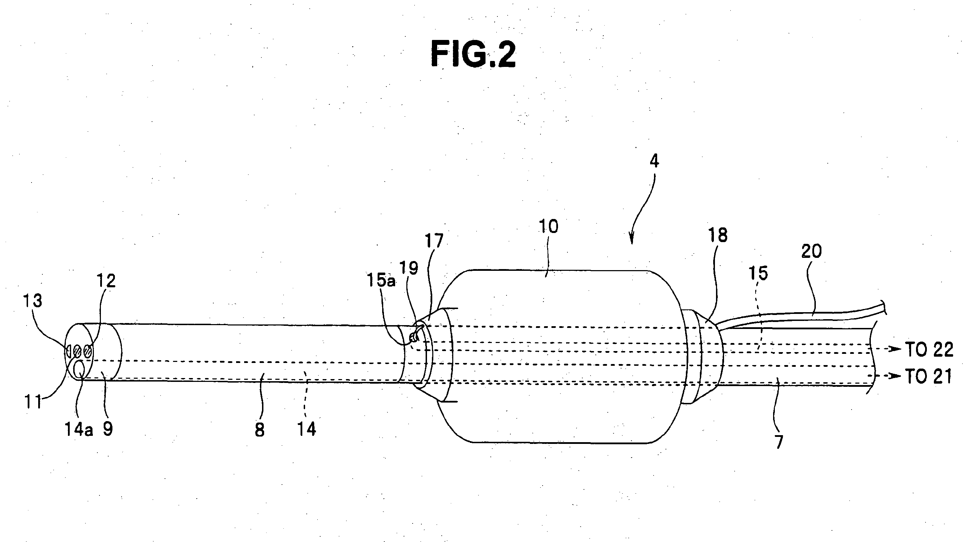

As shown in Fig. 2, an observation window 11 is provided near the center of a distal end face of the distal end portion 9, and an illumination window 12 and air/water supply nozzle 13 are provided on both sided of the observation window 11.

A light guide is arranged in the illumination window 12 to transmit illumination light via an illumination lens (not shown). The light guide is connected to the light source device by passing through the insertion portion 3 and the like. The illumination light generated by the light source device is transmitted through the light guide and emitted from the illumination window 12 to illuminate a field of view range of the observation window 11.

An objective lens (not shown) is arranged in the observation window 11 and an image pickup device such as a CCD is arranged at image forming position of the objective lens. An optical image of an interior of the intestinal tract is formed on an image pickup surface of the CCD.

A signal cable extends from the CCD. The signal cable is electrically connected to the signal processing device after passing through the insertion portion 3 and the like. The signal processing device outputs a CCD drive signal. Also, the signal processing device converts an image pickup signal produced by the CCD as a result of image pickup into a video signal and outputs the video signal to a monitor. Consequently, an image picked up by the CCD is displayed on a display screen of the monitor.

An opening portion 14a communicated with a distal opening of a first channel 14 is formed at a distal end of the distal end portion 9, where the first channel 14 provides a treatment instrument channel. Also, an opening of an opening portion 15a is formed in a rear flank of the bending portion 8 along an insertion direction. The opening portion 15a is communicated with a distal opening of a second channel 15 provided in the insertion portion 3.

The second channel 15 is provided to pass through a thread-like member 19 of the endoscope insertion aid 4.

The first channel 14 and second channel 15 are arranged along a longitudinal direction of the insertion portion 3. A proximal opening of the first channel 14 is communicated with a first channel entrance port 21 shown in Fig. 1 while a proximal opening of the second channel 15 is communicated with a second channel entrance port 22.

As shown in Fig. 1, the operation portion 5 has a grasping portion 23. The surgeon can operate a bending operation knob 24 in the operation portion 5 by gripping the grasping portion 23.

By rotating the bending operation knob 24, the surgeon can bend the bending portion 8 in a desired direction: up, down, left, or right.

The bending portion 8 is made up of multiple annular, bending pieces coupled rotatably and is designed to bend when the surgeon draws and relaxes a bending wire by rotating operation of the bending operation knob 24.

Also, as shown in Fig. 1, the operation portion 5 is provided with an air/water supply button 25 and a suction button 26 used for suction operations. By operating the air/water supply button 25, the surgeon can supply air or water. Also, by operating the suction button 26, the surgeon can suck body fluids and the like through the first channel 14 and its opening portion 14a.

The first channel 14 can be used as a suction conduit through which fluids are sucked and a conduit through which a treatment instrument is passed. For that, on the rear side, the first channel 14 is bifurcated into a conduit (not shown) which is communicated with the first channel entrance port 21 and a suction conduit (not shown) which extends to a rear end side of the operation portion 5.

Although in the present embodiment, the endoscope apparatus 1 has two channels--the first channel 14 and second channel 15, the endoscope apparatus 1 may have only the second channel 15. In that case, the second channel 15 can be used not only as a conduit through which the thread-like member 19 is passed, but also as a conduit through which a treatment instrument is passed and a suction conduit through which fluids are sucked as in the case of the first channel 14.

Now, a concrete configuration of the endoscope insertion aid 4 will be described.

As shown in Figs. 1, 2, 4, and 5, the endoscope insertion aid 4 includes a balloon tube member 16, balloon 10, thread-like member 19, fluid tube 20, distal-side entanglement prevention member 17, and rear-side entanglement prevention member 18.

The balloon tube member 16 is a tubular member which allows passage of the insertion portion 3 of the endoscope 2 and inserted insertion portion 3 moves forward and backward relatively along an insertion axis direction. The balloon 10 is a holding unit which is provided on an outer surface of the balloon tube member 16. The balloon 10 can be inflated and deflated as a fluid is supplied and discharged via the fluid tube 20. The thread-like member 19 is connected at one end to the balloon tube member 16. At the other end, the thread-like member 19 is passed into the second channel 15 through the opening portion 15a communicated with the second channel 15 of the insertion portion 3 and extends outward from the second channel entrance port 22 arranged on a proximal end side of the endoscope 2.

A thread-like member fixing unit (hereinafter abbreviated to a thread fixing unit) 30 is arranged on the proximal end side of the endoscope 2. The thread fixing unit 30 restrains the thread-like member 19 protruding from the second channel entrance port 22 and thereby restricts movement of the balloon tube member 16 toward the proximal end side with respect to the insertion portion 3 along the insertion axis. The fluid tube 20 doubles as a restraining member, being disposed in such a way as to be able to move relative to the insertion portion 3 along an axis.
The fluid tube 20 is connected at one end to the balloon tube member 16 and extends at the other end to the proximal end side along an outer peripheral side of the insertion portion 3. A tube/conduit member fixing unit (hereinafter abbreviated to a tube fixing unit) 31 holds the balloon tube member 16 at a desired position in the intestinal tract by fixing the above-described other end of the fluid tube 20.

Incidentally, the endoscope insertion aid 4 may be a disposable one which can be disposed of after a single use or a reusable one which can be reused by being cleaned, disinfected, and sterilized after use.

As shown in Fig. 2, the balloon 10 and balloon tube member 16 of the endoscope insertion aid 4 are arranged on the flexible tubular portion 7 closer to the proximal side along the insertion direction than the bending portion 8 so that the bending portion 8 will be exposed.

The thread-like member 19 connected to the balloon tube member 16 is passed into the second channel entrance port 22 through the opening portion 15a provided in the rear flank of the bending portion 8 along the insertion direction. In this way, the bending portion 8 is exposed without being covered by the balloon 10 to improve insertability into the intestinal tract by efficiently using bending operation of the bending portion 8.

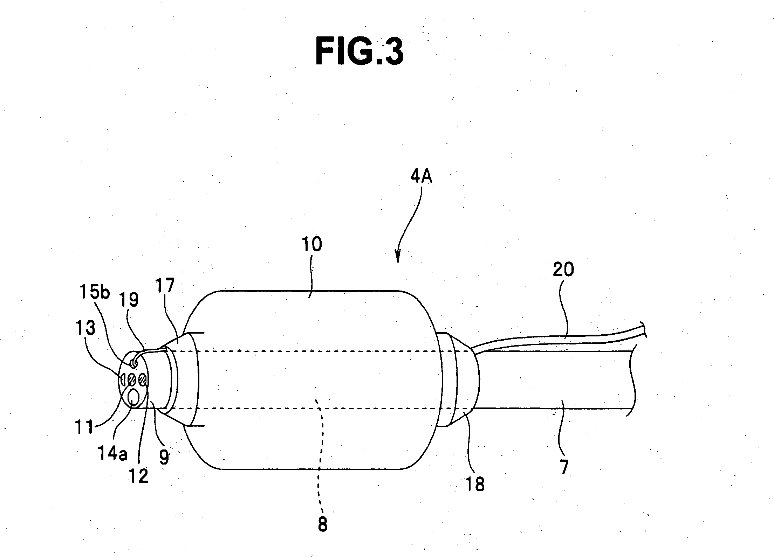

Incidentally, according to the present embodiment, the balloon 10 and balloon tube member 16 of the endoscope insertion aid 4 may be arranged on the distal side of the insertion portion 3 so as to cover the bending portion 8 as shown as a variation in Fig. 3.

In that case, the thread-like member 19 connected to the balloon tube member 16 is led to the second channel entrance port 22 after entering the opening portion 15b of the second channel 15 and passing through the second channel 15, where the opening portion 15b is an opening provided in a distal end face of the distal end portion 9.

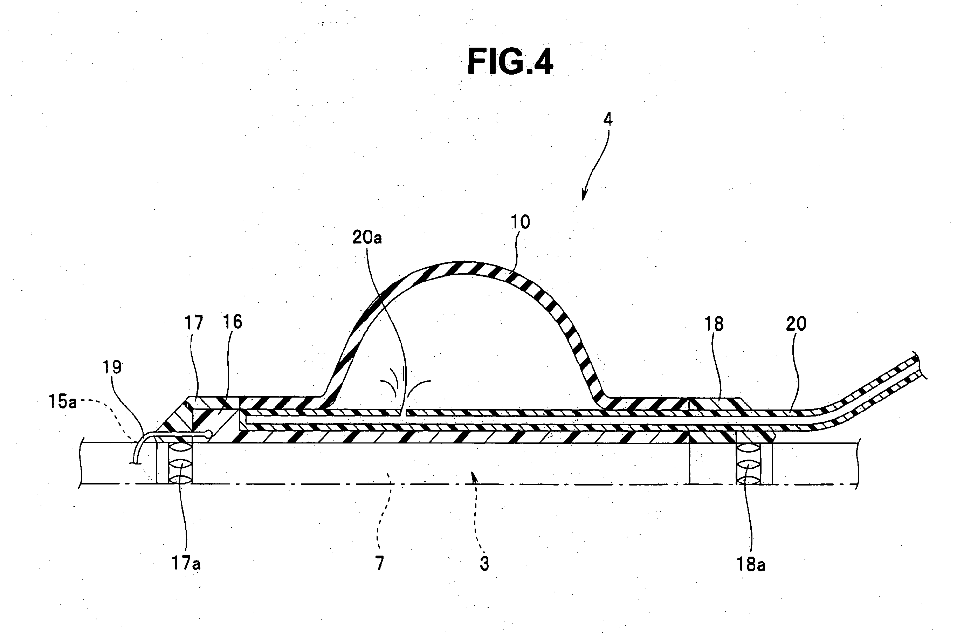

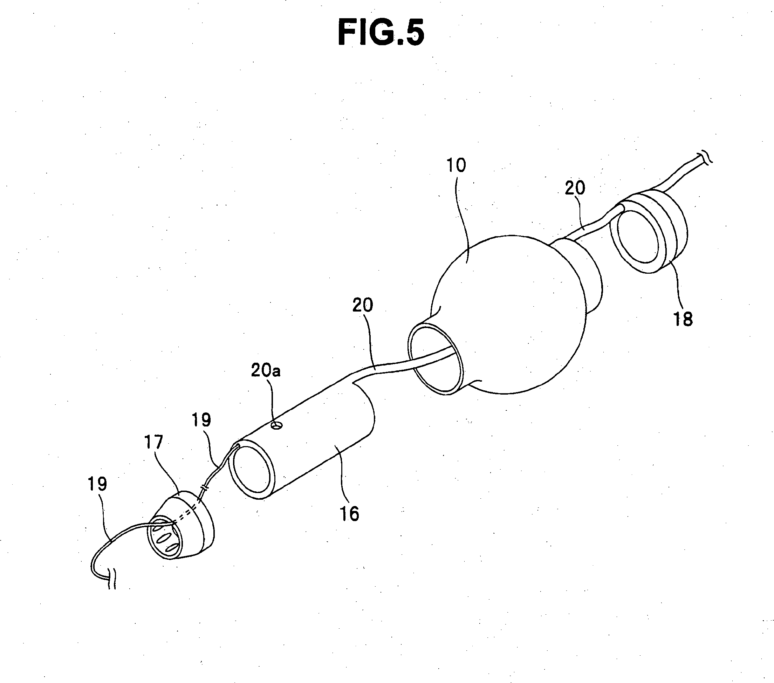

As shown in Figs. 4 and 5, the balloon 10 which inflates and deflates as a fluid is supplied and discharged is made of a highly elastic material such as silicon resin. When deflated, the balloon 10 assumes an substantially cylindrical shape.
A cylindrical inner peripheral surface of the balloon 10 is supported by the balloon tube member 16 on the distal side and rear side along the insertion direction.

The balloon tube member 16 is a flexible member made, for example, of silicon resin and formed into a cylindrical shape. The balloon tube member 16 is not limited particularly to any material or structure as long as the balloon tube member 16 has flexibility. For example, the balloon tube member 16 may be a fluoroplastic tube formed into a helical coil which is detachably fitted over the outer periphery of the flexible tubular portion 7, a fluoroplastic mesh tube, or a fluoroplastic tube harder and less elastic than the balloon 10.

The fluid tube 20 which is an elongated tube member is disposed in the balloon tube member 16 to supply and discharge the fluid to the balloon 10. Also, the fluid tube 20 has an opening 20a for use to supply and discharge the fluid. The opening 20a is formed at a location which is communicated with a hollow portion in the balloon 10. The fluid tube 20 is made, for example, of silicon.

Incidentally, the balloon tube member 16 and fluid tube 20 may be formed integrally or formed as separate members as shown in Fig. 4.

The fluid tube 20, which is configured to be flexible, preferably has such strength and characteristics that the fluid tube 20 inserted in the intestinal tract together with the insertion portion 3 can be drawn by the surgeon toward the user's hand side, with the fluid tube 20 being held in the intestinal tract, for example, by the inflated balloon 10.

As shown in Fig. 1, a rear end of the fluid tube 20 is detachably and airtightly connected to a front end of a tube 34 connected to a balloon control pump 33, which is an example of fluid supply/discharge means.

Operation of the balloon control pump 33 can be controlled by turning on and off a balloon control switch (not shown). By operating the balloon control switch, the surgeon can freely supply a gas such as air from the balloon control pump 33 into the balloon 10 through the fluid tube 20 and thereby inflate the balloon 10 or suck or discharge the fluid from the balloon 10 and thereby deflate the balloon 10. Incidentally, the fluid such as air may be supplied and discharged manually using a syringe or the like instead of the balloon control pump 33.

As shown in Figs. 4 and 5, the distal-side entanglement prevention member 17 is fixed to a distal end portion of the balloon tube member 16. The distal-side entanglement prevention member 17, which has a tubular shape, is tapered toward the distal end, forming a tapered portion on the distal side. Also, the rear-side entanglement prevention member 18 is fixed to a rear end portion of the balloon tube member 16. Again, the rear-side entanglement prevention member 18, which has a tubular shape, is tapered toward the rear end, forming a tapered portion on the rear side.

Multiple protrusions 17a are provided in a circumferential direction on an inner peripheral surface of the distal-side entanglement prevention member 17 to reduce a contact area with the insertion portion 3 and thereby reduce friction. Also, multiple protrusions 18a are provided on an inner peripheral surface of the rear-side entanglement prevention member 18 to reduce a contact area with the insertion portion 3 and thereby reduce friction.

That is, since a clearance from the insertion portion 3 is reduced by the multiple protrusions 17a and 18a, the distal-side entanglement prevention member 17 and rear-side entanglement prevention member 18 can prevent intestinal walls and the like from being entangled when the insertion portion 3 advances into the intestinal tract together with the balloon 10 and balloon tube member 16 or when the balloon 10 and balloon tube member 16 are moved toward the user's hand side along the insertion axis of the insertion portion 3.

Incidentally, the distal-side entanglement prevention member 17 is provided with a passage hole to pass the thread-like member 19. Also, the rear-side entanglement prevention member 18 is provided with a passage hole to pass the fluid tube 20 along the insertion axis.

As shown in Fig. 4, one end of the thread-like member 19 is fixed to the distal side of the balloon tube member 16. There is no particular limit to a method for fixing the end of the thread-like member 19 to the balloon tube member 16. For example, the end of the thread-like member 19 is fixed with an adhesive or the like in a mounting hole (not shown) provided in a distal end face of the balloon tube member 16 or the end of the thread-like member 19 is fixed with an adhesive or the like to a groove provided in an inner peripheral surface of the balloon tube member 16.

As shown in Fig. 2, the thread-like member 19 is passed through the distal-side entanglement prevention member 17, and then passed into the second channel 15 of the insertion portion 3 through the opening portion 15a provided in the rear flank of the bending portion 8 along the insertion direction. Then, the other end of the thread-like member 19 is extended outward from the second channel entrance port 22 of the endoscope 2 as shown in Fig. 1.

The thread-like member 19, which is configured to be flexible, preferably has such strength and characteristics as not to break even if loads are exerted on the balloon 10 and balloon tube member 16 during insertion into the intestinal tract.

Now, a method for assembling components of the endoscope insertion aid 4 will be described.

As shown in Fig. 5, the balloon 10 is fitted in a predetermined position of the balloon tube member 16 which includes the fluid tube 20 and inner peripheral surface of the balloon 10 is fixed, on the distal side and proximal side, to an outer peripheral surface of the balloon tube member 16 with an adhesive or the like.

Next, one end of the thread-like member 19 is fixed to the balloon tube member 16. Then, the thread-like member 19 is passed through the distal-side entanglement prevention member 17. In this state, the distal-side entanglement prevention member 17 is fixed to the distal end portion of the balloon tube member 16, for example, with an adhesive or the like.

On the other hand, the fluid tube 20 extending from the rear side of the balloon tube member 16 is passed into the passage hole of the rear-side entanglement prevention member 18, which is then fixed to a rear end portion of the balloon tube member 16, for example, with an adhesive or the like.

Incidentally, the method for fixing the balloon 10, distal-side entanglement prevention member 17, and rear-side entanglement prevention member 18 to the balloon tube member 16 is not limited to the use of an adhesive or the like, and another fixing method may be used.

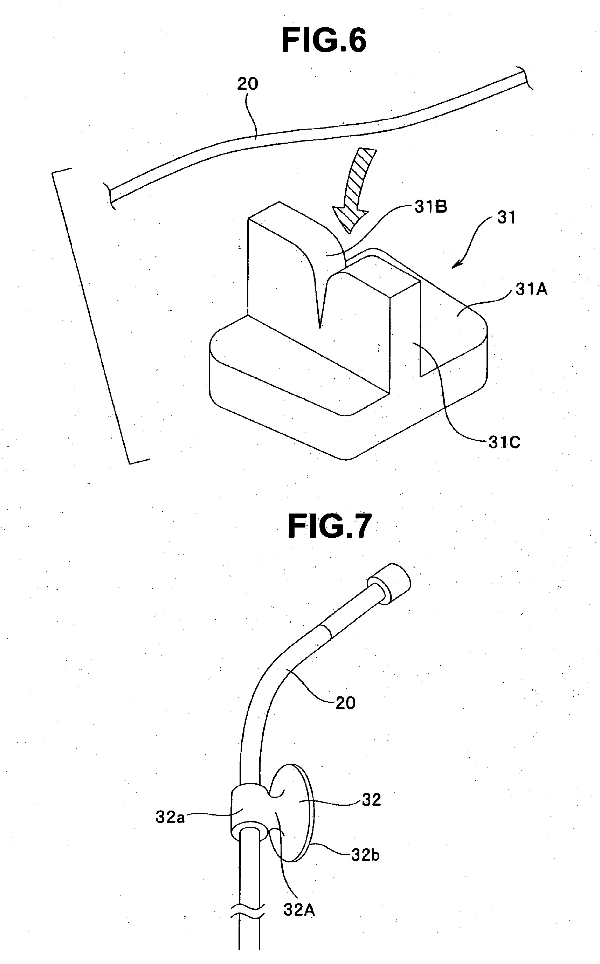

Next, configuration of the thread fixing unit 30 and tube fixing unit 31 of the endoscope insertion aid 4 will be described with reference to Figs. 6 to 9.

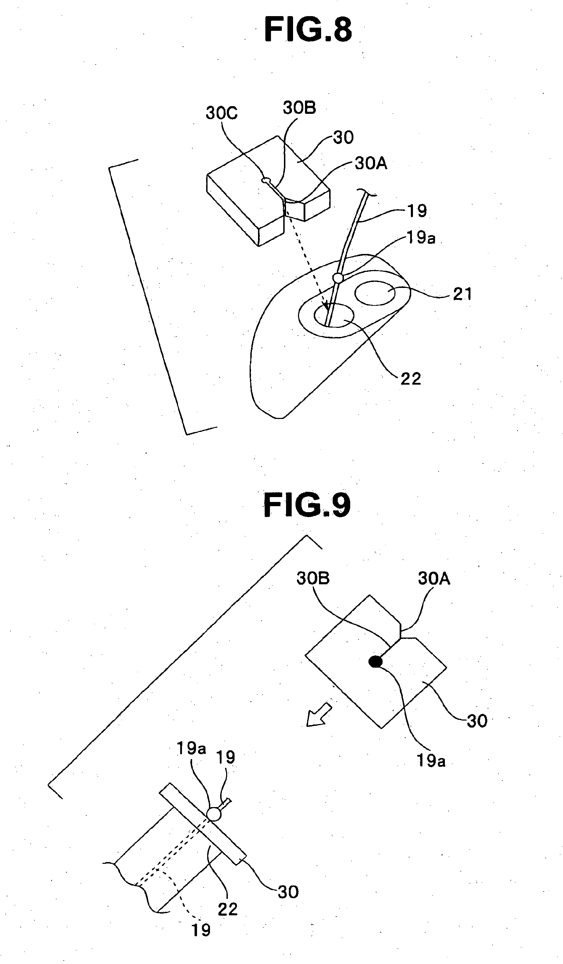

A main body of the thread fixing unit 30 shown in Figs. 1 and 8 is made, for example, of resin and structured as a rectangular solid. Besides, the thread fixing unit 30 is provided with a notch 30A having a tapered space between surfaces thereof, slit 30B, and passage hole 30C. The notch 30A is formed in a flank of the main body and the slit 30B extends from the notch 30A. The passage hole 30C forms an end portion of the slit 30B, being located in the approximate center of the main body.

The passage hole 30C is designed to be large enough to pass the thread-like member 19, but not large enough to pass a stopper 19a provided at a predetermined position of the thread-like member 19.

Thus, to restrict movement of the balloon tube member 16 with respect to the insertion portion 3 using the thread fixing unit 30, the surgeon provides the stopper 19a at a predetermined position of the thread-like member 19 in advance. Then, as shown in Fig. 8, the surgeon places the thread-like member 19 protruding from the second channel entrance port 22 in contact with the notch 30A in the thread fixing unit 30 and pushes the thread-like member 19 into the slit 30B.

Then, as shown in Fig. 9, once the thread-like member 19 is arranged in the passage hole 30C, the surgeon brings the thread fixing unit 30 into abutment against an opening portion of the second channel entrance port 22. Consequently, the stopper 19a of the thread-like member 19 is restrained around the passage hole 30C.

This makes it possible to restrict movement of the balloon tube member 16 toward the proximal end side along the insertion axis of the insertion portion 3.

Incidentally, position of the stopper 19a with respect to the thread-like member 19 can be changed as required. By changing the position of the stopper 19a, it is possible to change restricting position of the balloon tube member 16 with respect to the insertion portion 3.

With the thread-like member 19 pulled in a direction away from the second channel entrance port 22, the surgeon can remove the thread fixing unit 30 from the thread-like member 19 by reversing the fixing operation described above. The removal of the thread fixing unit 30 from the thread-like member 19 derestricts the movement of the balloon tube member 16 toward the proximal end side with respect to the insertion portion 3 along the insertion axis.

On the other hand, the tube fixing unit 31 shown in Fig. 6 includes a pedestal body 31A and a restraining portion 31C provided with a V groove 31B. The V groove 31B is erected on a flat surface of the pedestal body 31A to restrain and hold the fluid tube 20.

The tube fixing unit 31 is placed on or near a patient bed. Therefore, it is preferable that the tube fixing unit 31 is heavy enough not to move easily. If the tube fixing unit 31 is not heavy enough, preferably the tube fixing unit 31 is fixed to or near the patient bed so as not to move easily.

Thus, to fix the balloon tube member 16 at a desired position in the intestinal tract using the tube fixing unit 31, the surgeon pushes the fluid tube 20 into the V groove 31B of the tube fixing unit 31. Consequently, the fluid tube 20 is slightly squeezed by the V groove 31B and thereby fixed in the V groove 31B securely.

This makes it possible to hold the balloon tube member 16 at the desired position in the intestinal tract.

Incidentally, the configuration of the tube fixing unit 31 is not limited to the one shown in Fig. 6, and any other configuration may be used as long as the fluid tube 20 can be fixed securely at a desired position.

Also, the surgeon can release the balloon tube member 16 held at the desired position in the intestinal tract by removing the fluid tube 20 from the V groove 31B of the tube fixing unit 31.

Also, according to the present embodiment, the fluid tube 20 is provided with a fixing unit 32 as shown in Fig. 7. The fixing unit 32 fixes and holds the fluid tube 20 to or near a patient bed. The fixing unit 32 includes a main body 32A, a holding unit 32a which, being provided integrally with the main body 32A, allows passage of the fluid tube 20, and a suction cup unit 32b provided on a bottom surface of the main body 32A.

The suction cup unit 32b is configured to be fixed to a planar member by suction, but the fixing unit 32 is not limited to a configuration in which the main body 32A has the suction cup unit 32b, and may be configured, for example, to be an attachable/detachable adhesive member.

Also, the configuration of the fixing unit 32 is not limited to the one shown in Fig. 7, and any other configuration may be used as long as the fluid tube 20 can be fixed at a desired position.

In this way, by restraining the thread-like member 19 with the thread fixing unit 30, the endoscope apparatus 1 according to the present embodiment can restrict the movement of the balloon tube member 16 of the endoscope insertion aid 4 toward the proximal end side with respect to the insertion portion 3 along the insertion axis. On the other hand, by releasing the thread-like member 19 from the latching of the thread fixing unit 30, the endoscope apparatus 1 enables the movement of the balloon tube member 16 toward the proximal end side with respect to the insertion portion 3 along the insertion axis.

That is, the endoscope apparatus 1 is configured such that when the movement of the balloon tube member 16 is restricted, it becomes easier to insert the insertion portion 3 together with the balloon tube member 16 of the endoscope insertion aid 4 into the intestinal tract. On the other hand, when the movement of the balloon tube member 16 is derestricted, it becomes easier to move the insertion portion 3 forward reliably with respect to the balloon tube member 16.

Also, the endoscope apparatus 1 is configured such that the balloon tube member 16 can be held at a desired position in the intestinal tract as the fluid tube 20 is fixed with the tube fixing unit 31 and that the balloon tube member 16 can be moved in the intestinal tract as the fluid tube 20 is released from the tube fixing unit 31.

That is, the endoscope apparatus 1 is configured such that when the fluid tube 20 is unfixed, allowing the fluid tube 20 to be drawn toward the user's hand side, the intestinal tract held by the inflated balloon 10 can be hauled in easily. On the other hand, when the fluid tube 20 is fixed, holding the intestinal tract in the hauled state, it becomes easy to straighten the intestinal tract and thereby help insert the endoscope.

Incidentally, although according to the present embodiment, the thread-like member 19 and thread fixing unit 30 are used to restrict and derestrict the movement of the balloon tube member 16 toward the proximal end side with respect to the insertion portion 3 along the insertion axis, the method for restricting and derestricting the movement of the balloon tube member 16 is not limited thereto.
For example, a separate inner balloon may be provided on an inner peripheral side of the balloon tube member 16 to restrict and derestrict the movement of the balloon tube member 16 relative to the insertion portion 3 by inflating and deflating the inner balloon, as with an embodiment described later.

Also, the surgeon may restrain and release the thread-like member 19 to/from the second channel entrance port 22 with fingers without using the thread fixing unit 30. Besides, the thread fixing unit 30 may be configured to be able to mechanically draw, restrain, and release the thread-like member 19, as with a variation of the thread fixing unit 30 described later. In that case, the thread-like member 19 may be drawn, restrained, and released electrically.

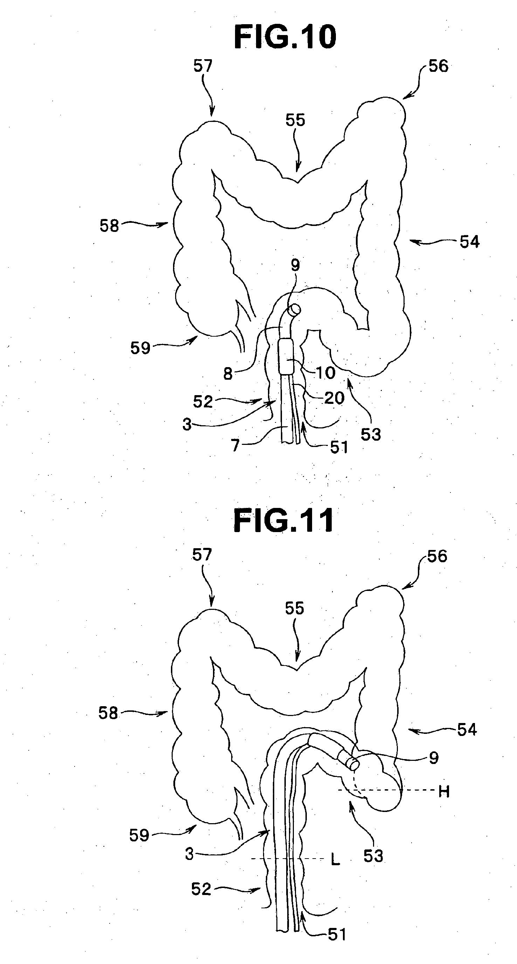

Next, with reference to Figs. 10 to 15, description will be given of how the endoscope 2 according to the present embodiment configured as described above is inserted in the intestinal tract to perform endoscopy.

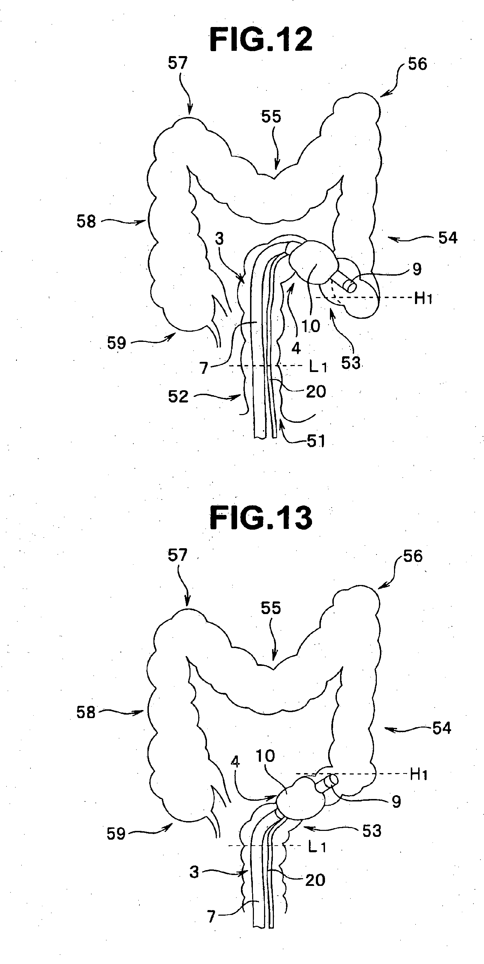

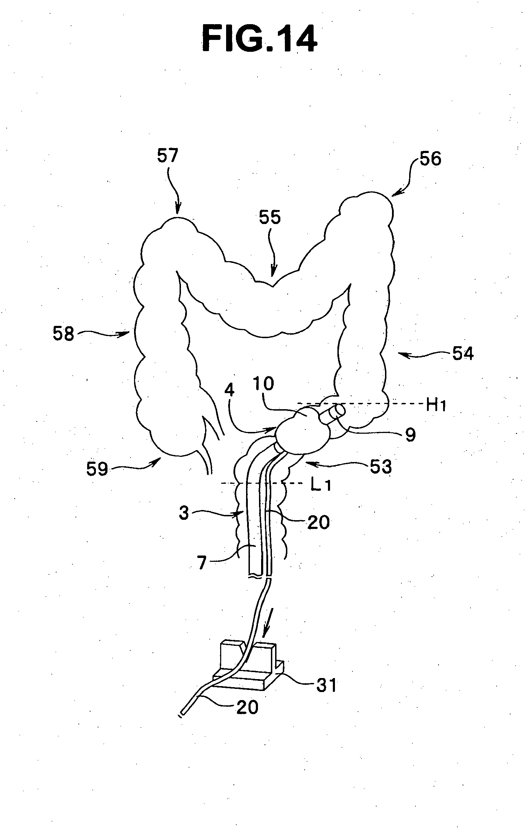

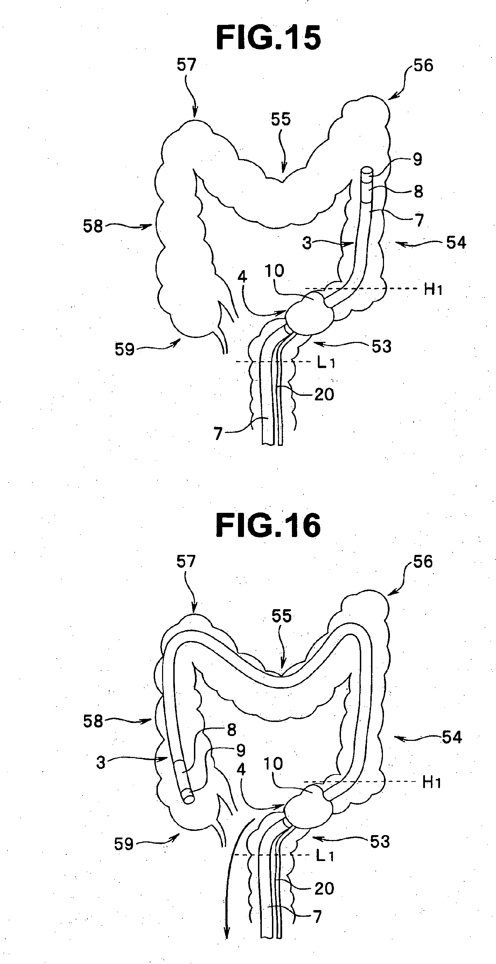

Figs. 10 to 15 are explanatory diagrams illustrating the operation of inserting the insertion portion 3 using the endoscope insertion aid 4 according to the first embodiment, where Fig. 10 is a diagram showing how the insertion portion is inserted through the anus with the balloon tube member fixed; Fig. 11 is a diagram showing how the insertion portion is inserted from the rectum to the sigmoid colon portion by bending operation and the like from a state shown in Fig. 10; Fig. 12 is a diagram showing how the sigmoid colon portion is held by inflating the balloon in a state shown in Fig. 11; Fig. 13 is a diagram showing how the intestinal tract is hauled in and straightened by drawing the insertion portion and fluid tube in a state shown in Fig. 12; Fig. 14 is a diagram showing how the balloon tube member is locked by holding the fluid tube using the tube fixing member in a state shown in Fig. 13; and Fig. 15 is a diagram showing how the insertion portion is inserted further into the splenic flexure in a deep part of the intestinal tract by releasing the thread-like member from the thread fixing unit in a state shown in Fig. 14.

Before endoscopy, the thread-like member 19 connected to the balloon tube member 16 is passed into the second channel 15 through the opening portion 15a of the second channel 15 provided in the rear flank of the bending portion 8 along the insertion direction as shown in Fig. 2 and is led out of the second channel entrance port 22 as shown in Fig. 1. Also, as shown in Fig. 2, the endoscope insertion aid 4 is fitted over a distal outer peripheral surface, i.e., an outer peripheral surface of the flexible tubular portion 7 on the rear side of the bending portion 8, so that the bending portion 8 of the endoscope 2 will be exposed.

In this state, to maintain the position of the endoscope insertion aid 4 with respect to the insertion portion 3, the thread-like member 19 led out of the second channel entrance port 22 is held by the thread fixing unit 30, thereby restricting the movement of the balloon tube member 16 with respect to the insertion portion 3.

Also, on the rear side, the fluid tube 20 of the endoscope insertion aid 4 extends to the user's hand side of the insertion portion 3. This allows the surgeon to grip and draw the fluid tube 20.

To perform endoscopy in the intestinal tract, the surgeon inserts the endoscope 2, starting from the distal side, into the intestinal tract with the balloon 10 deflated as shown in Fig. 10.

In so doing, the surgeon inserts a distal end portion 9 of the insertion portion 3 of the endoscope 2 through the anus 51 into the rectum 52 and further toward the sigmoid colon portion 53 using hand manipulation, bending operation, and the like.

Then, as shown in Fig. 11, the distal end portion 9 of the endoscope 2 approaches the sigmoid colon portion 53 into which it is difficult to insert the insertion portion 3. When the insertion portion 3 is pushed in, since a bent portion between the rectum 52 and sigmoid colon portion 53 has high mobility, the insertion portion 3 is pushed further into the intestinal tract, causing a bent portion to be formed between the sigmoid colon portion 53 and the descending colon portion 54 which has low mobility.

Then, in a state shown in Fig. 11, the surgeon turns on the balloon control switch (not shown) to drive the balloon control pump 33. Consequently, the balloon control pump 33 supplies a fluid such as air into the balloon 10 via the fluid tube 20, thereby starting to inflate the balloon 10.

Then, as shown in Fig. 12, the balloon 10 inflates and holds the sigmoid colon portion 53, a part of the intestinal tract, from inside.

As shown in Fig. 12, with the sigmoid colon portion 53 held by inflation of the balloon 10, the surgeon draws the insertion portion 3 and fluid tube 20 slowly and thereby hauls in the intestinal tract toward the user's hand side.

Consequently, as shown in Fig. 13, as the surgeon draws the fluid tube 20 and thereby hauls in the intestinal tract held by the balloon 10, the bent portion formed at the junction between the sigmoid colon portion 53 and descending colon portion 54 is extended. That is, the sigmoid colon portion 53 is straightened between the junction H1 with the descending colon portion 54 and a junction L1 with the rectum 52.

Then, as shown in Fig. 14, the surgeon fixes the fluid tube by pushing the fluid tube 20 in the V groove 31 B of the tube fixing unit 31 fixed to the patient bed or the like.

This makes it possible to hold the balloon 10 and balloon tube member 16 in the sigmoid colon portion 53 straightened as shown in Fig. 14. Also, since the fluid tube 20 is fixed to the tube fixing unit 31, the hauled intestinal tract does not return to its original state.

In a state shown in Fig. 14, the surgeon removes the thread fixing unit 30 holding the thread-like member 19 and thereby derestricts the movement of the endoscope insertion aid 4 with respect to the insertion portion 3.

Next, with the sigmoid colon portion 53 kept straightened by the balloon 10, the surgeon inserts the insertion portion 3 movable with respect to the endoscope insertion aid 4 into a deeper part. Consequently, the insertion portion 3 is inserted through the descending colon portion 54 toward the splenic flexure 56 located at the junction between the descending colon portion 54 and the transverse colon portion 55 which has high mobility.

In this way, insertion operation assisted by the endoscope insertion aid 4 makes it easy to straighten the sigmoid colon portion 53 into which insertion is difficult. By straightening the sigmoid colon portion 53, it becomes easy to advance the insertion portion 3 deep into the intestinal tract by passing through the sigmoid colon portion 53.

Then, to insert the insertion portion 3 deeper into the intestinal tract, the surgeon deflates the balloon 10, draws the thread-like member 19 slowly, and thereby moves the balloon tube member 16 to the distal side of the insertion portion 3.

Then, in the manner described above, the surgeon holds the thread-like member 19 using the thread fixing unit 30, thereby fixes the balloon tube member 16 with respect to the insertion portion 3, and inserts the insertion portion 3 again, using hand manipulation, bending operation, and the like, into the splenic flexure 56 located at the junction between the descending colon portion 54 and the transverse colon portion 55 which has high mobility.

Subsequently, by repeating the straightening operation described above, the surgeon can insert the distal end portion 9 of the insertion portion 3 into a deep part of the intestinal tract near the cecum portion 59 by passing through the hepatic flexure 57 located at the junction between the transverse colon portion 55 and ascending colon 58.

Alternatively, as shown in Fig. 15, the surgeon may insert the insertion portion 3 until the insertion portion 3 approaches the cecum portion 59, with the sigmoid colon portion 53 held and straightened by the balloon 10.

Next, a method for pulling out the insertion portion 3 after endoscopy will be described with reference to Figs. 16 to 18.

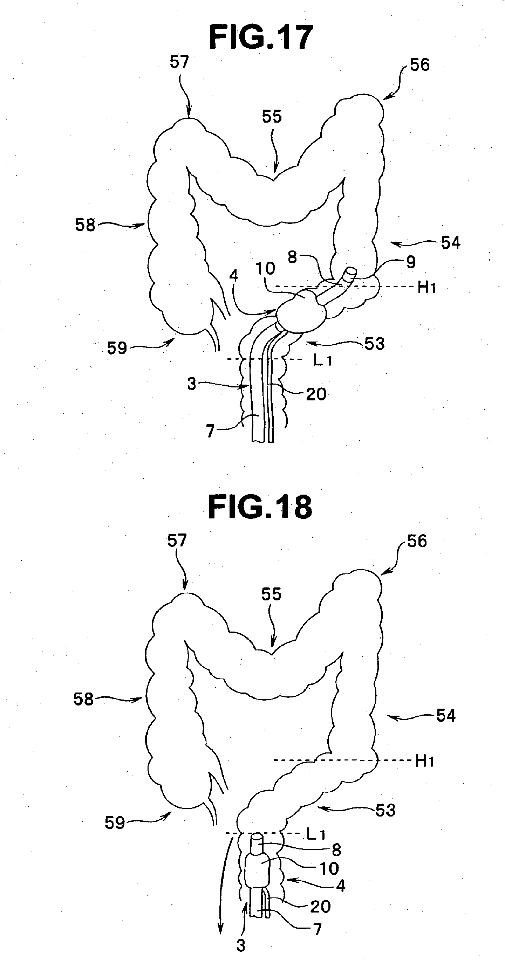

Figs. 16 to 18 are explanatory diagrams illustrating the operation of pulling out the insertion portion 3 using the endoscope insertion aid 4 according to the first embodiment, where Fig. 16 is a diagram showing how the distal side of the insertion portion 3 has reached the cecum portion in a state shown in Fig. 15; Fig. 17 is a diagram showing how the insertion portion is drawn by releasing the fluid tube from the tube fixing member in the state shown in Fig. 15; and Fig. 17 is a diagram showing how the insertion portion and fluid tube are drawn and pulled out of the anus by deflating the balloon in a state shown in Fig. 16.

After the endoscopy, in the state shown in Fig. 15, i.e., with the thread fixing unit 30 removed which held the thread-like member 19 and with the movement of the endoscope insertion aid 4 with respect to the insertion portion 3 derestricted, the surgeon draws the insertion portion 3 slowly in the direction indicated by an arrow in Fig. 16 while keeping the sigmoid colon portion 53 straightened by the balloon 10.

When the distal side of the insertion portion 3 approaches the junction between the descending colon portion 54 sigmoid colon portion 53 as shown in Fig. 17, the surgeon deflates the balloon 10 and releases the sigmoid colon portion 53 from the balloon 10.

The surgeon restricts the movement of the balloon tube member 16 with respect to the insertion portion 3 by restraining the thread-like member 19 with the thread fixing unit 30 again and draws the fluid tube 20 together with the insertion portion 3.

As shown in Fig. 18 this makes it easy to pull out the insertion portion 3 of the endoscope 2 together with the endoscope insertion aid 4 through the rectum 52 and anus 51.

As described above, according to the first embodiment, the endoscope insertion aid 4 includes the thread fixing unit 30 which holds the thread-like member 19 and thereby restricts the movement of the balloon tube member 16 toward the proximal end side with respect to the insertion portion 3 along the insertion axis, and the tube fixing unit 31 which fixes the fluid tube 20 and thereby holds the balloon tube member 16 at a desired position in the intestinal tract, in addition to the balloon 10, balloon tube member 16, thread-like member 19, and fluid tube 20. Consequently, after the intestinal tract held by the balloon 10 is hauled in by drawing the fluid tube 20, the state of the intestinal tract can be maintained using a simple configuration and operation method. This makes it possible to easily straighten the intestinal tract and smoothly insert the insertion portion 3 of the endoscope 2 deep into the intestinal tract.

Incidentally, although according to the present embodiment, the insertion portion 3 of the endoscope 2 is inserted into the large intestine with the help of the endoscope insertion aid 4, the lumen into which the insertion portion 3 equipped with the endoscope insertion aid 4 is inserted is not limited to the large intestine, and may be the lumen running from oral cavity, the esophagus, the stomach and to the small intestine.

Also, according to the present embodiment, the thread fixing unit 30 which has a simple rectangular body is configured to restrain and release the thread-like member 19. However, in addition to being able to restrain and release the thread-like member 19, the thread fixing unit may be configured such as allow the surgeon to adjust an amount of movement of the balloon tube member 16 with respect to the insertion portion 3 during a return operation in which the thread-like member 19 is drawn and perform the return operation smoothly, as shown in an embodiment described later.

### (Second embodiment)

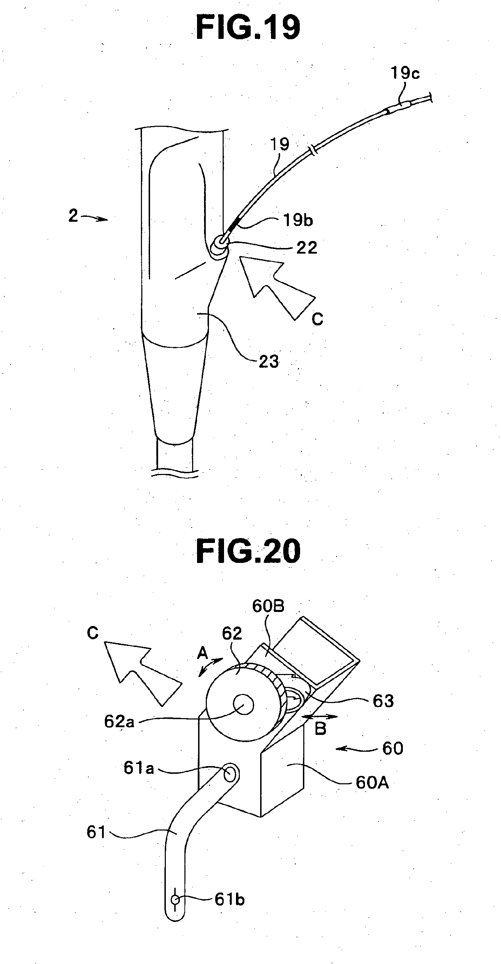

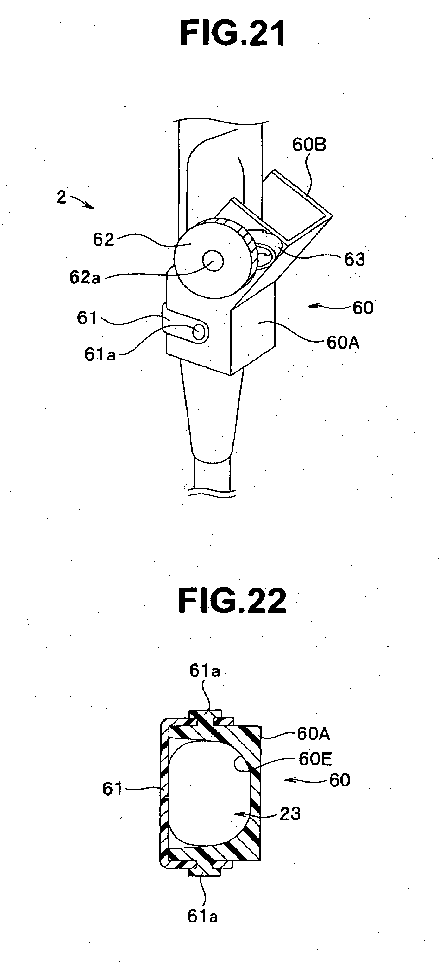

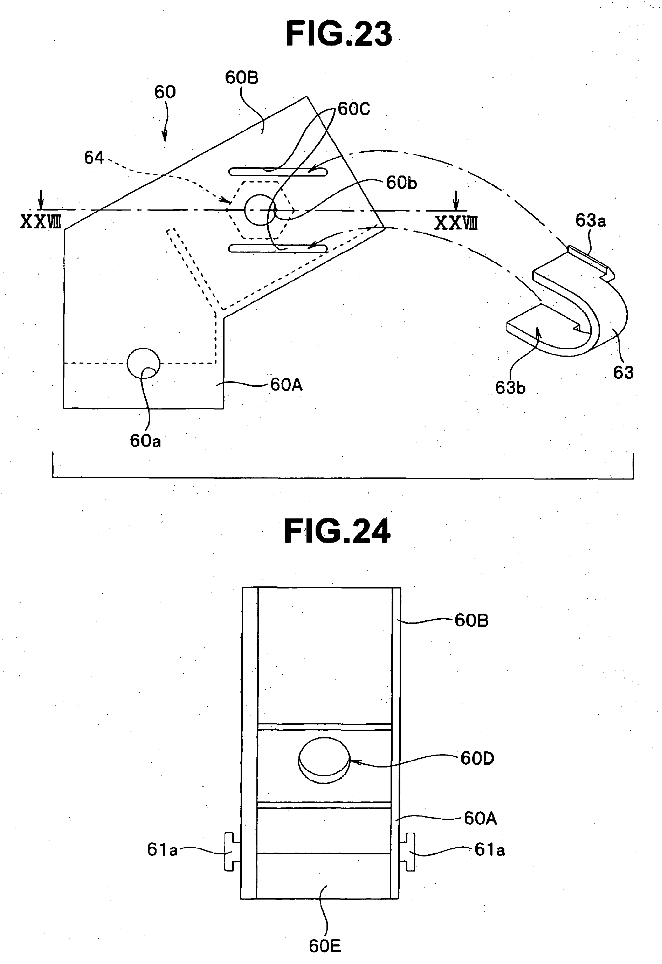

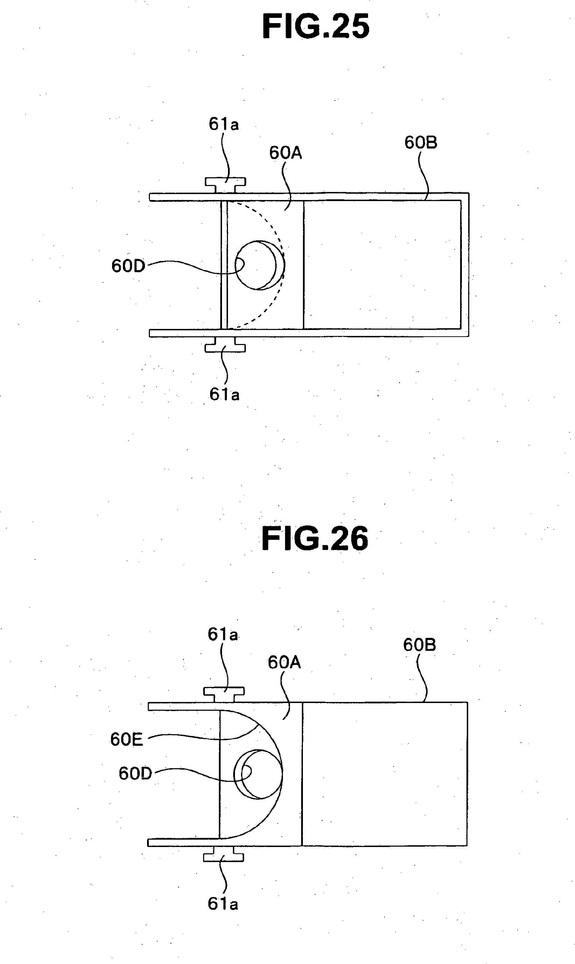

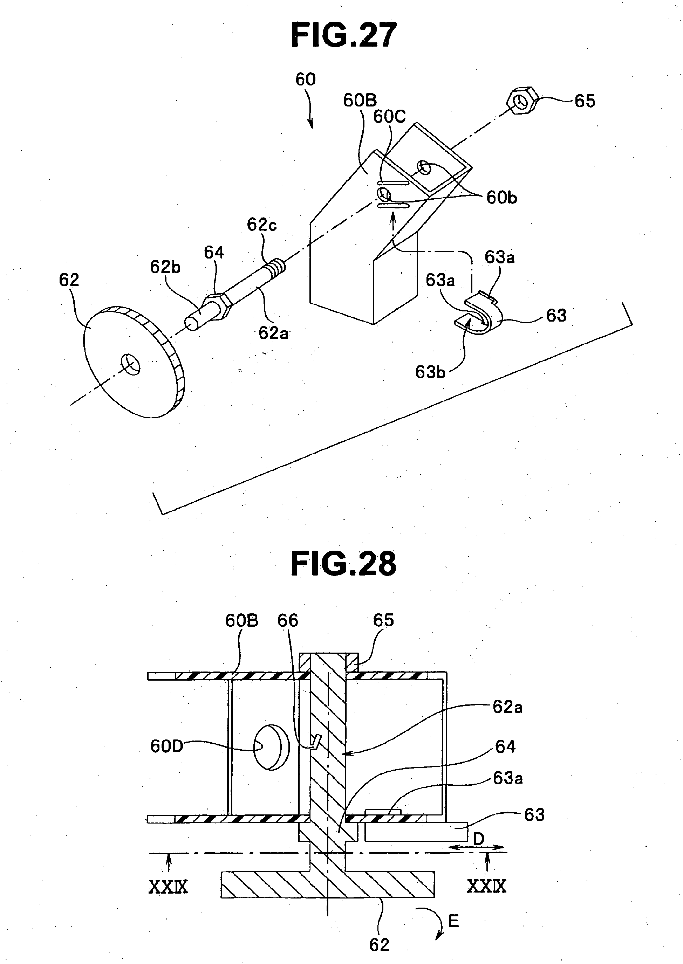

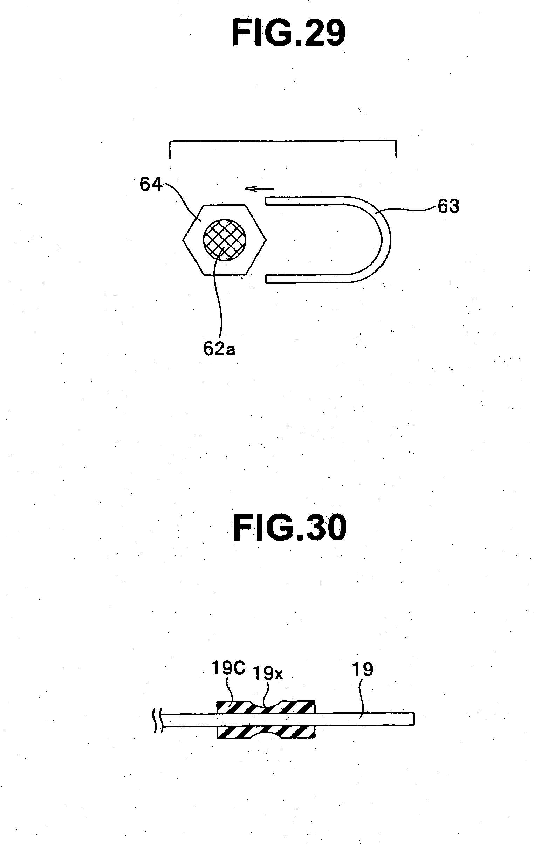

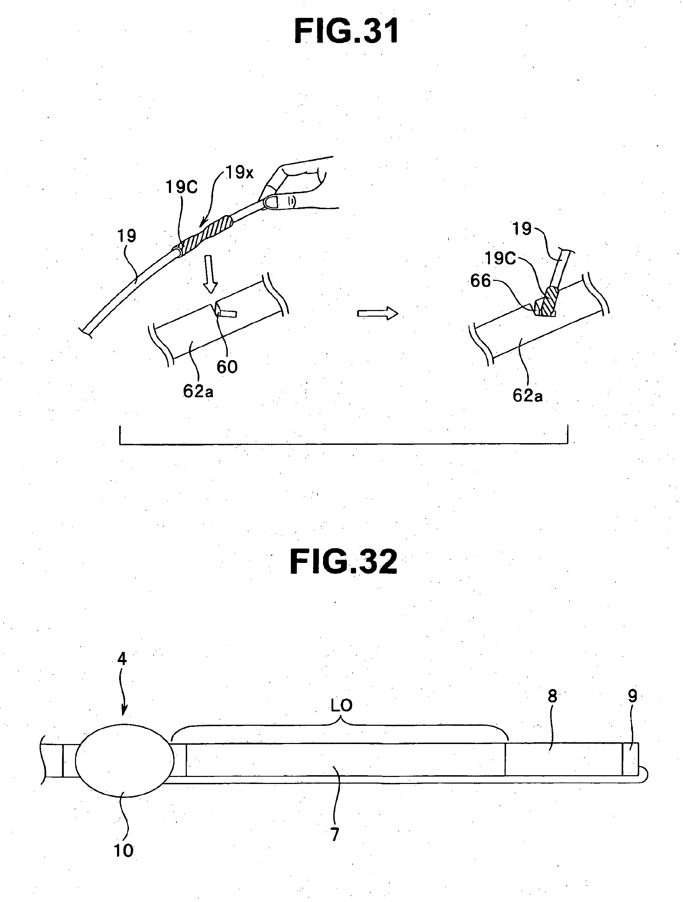

Figs. 19 to 31 concern a second embodiment, where Fig. 19 is an explanatory diagram showing how an improved thread-like member is led out of a second channel entrance port; Fig. 20 is a perspective view illustrating a configuration of an improved rotary-dial thread fixing unit; Fig. 21 is a perspective view showing how the rotary-dial thread fixing unit shown in Fig. 20 is mounted near the second channel entrance port of the endoscope; Fig. 22 is a sectional view of a mounting portion of the endoscope and rotary-dial thread fixing unit shown in Fig. 21; Fig. 23 is an exploded block diagram showing a concrete configuration of the rotary-dial thread fixing unit shown in Fig. 20; Fig. 24 is a block diagram of the rotary-dial thread fixing unit shown in Fig. 23 as viewed from a mounting direction of the endoscope; Fig. 25 is a block diagram of the rotary-dial thread fixing unit shown in Fig. 23 as viewed from above; Fig. 26 is a block diagram of the rotary-dial thread fixing unit shown in Fig. 23 as viewed from below; Fig. 27 is an exploded perspective view illustrating a configuration of principal part of the rotary-dial thread fixing unit shown in Fig. 20; Fig. 28 is a sectional view taken along line XXVIII - XXVIII in Fig. 23; Fig. 29 is a sectional view taken along line XXIX - XXIX in Fig. 28; Fig. 30 is a sectional view near an elastic portion of the thread-like member used in the present embodiment; and Fig. 31 is an explanatory diagram illustrating the operation of restraining the elastic portion of the thread-like member in a grooved portion of a rotating shaft of the thread fixing unit.

As shown in Figs. 19 and 20, an endoscope apparatus 1 according to the present embodiment has a thread-like member 19 improved over the one according to the first embodiment and includes a rotary-dial thread fixing unit 60 instead of the thread fixing unit 30 according to the first embodiment.

The thread-like member 19 used in the present embodiment includes an elastic portion 19c and marker 19b. The elastic portion 19c is made of an elastic material and provided on a proximal side of the thread-like member 19. The elastic portion 19c and the marker 19b is provided closer to the distal side a predetermined distance away from the elastic portion 19c.

The elastic portion 19c covers an outer surface of the thread-like member 19, for example, as shown in Fig. 30. A recessed portion 19x is provided near the longitudinal center of the elastic portion 19c to make it easy to restrain the elastic portion 19c in a groove 66 formed in a rotating shaft 62a shown in Fig. 31.

The marker 19b, which is intended to be used by the surgeon for the purpose of identification, is created by applying a paint or colored tape to the outer surface of the thread-like member 19.

The rotary-dial thread fixing unit 60 according to the present embodiment is configured to be able to restrain the thread-like member 19 configured as described above, hold the balloon tube member 16 by winding the thread-like member 19, and release the thread-like member 19 from the restraint.

As shown in Fig. 19, the rotary-dial thread fixing unit 60 is detachably mounted on a predetermined portion of a grasping portion 23 in which the second channel entrance port 22 of the endoscope 2 is provided. The rotary-dial thread fixing unit 60 is mounted in such a way as to cover the second channel entrance port 22 along arrow C shown in Figs. 19 and 20.

Incidentally, although the endoscope 2 according to the present embodiment has only the second channel entrance port 22 as shown in Fig. 19, the endoscope 2 may have two channel entrance ports 21 and 22 as with the first embodiment. In that case, the rotary-dial thread fixing unit 60 needs to be configured according to its shape.

Now, a concrete configuration of the rotary-dial thread fixing unit 60 will be described with reference to Figs. 20 to 29.

As shown in Fig. 20, the rotary-dial thread fixing unit 60 mainly includes a main body 60A, fitting unit 60B, mounting belt 61, rotary dial 62, and slide lock 63. The main body 60A is detachably fitted over a lower part of the grasping portion 23 of the endoscope 2. The fitting unit 60B, which is attached to the main body 60A, is fitted over the grasping portion 23 near the second channel entrance port 22 in such a way as to cover the second channel entrance port 22. The mounting belt 61 is used to detachably mount the main body 60A on the grasping portion 23. The rotary dial 62 is used to wind the thread-like member 19 by being provided on the fitting unit 60B in such a way as to be rotatable in a direction of arrow A. The slide lock 63, which is slidable with respect to the fitting unit 60B as indicated by arrow B, is used to restrict and derestrict rotation of the rotary dial 62.

The main body 60A and fitting unit 60B are configured to be, for example, substantially channel-shaped as shown in Figs. 20 to 22 so that the main body 60A and fitting unit 60B can be fitted over the grasping portion 23 at a location near the second channel entrance port 22. A contact portion 60E is formed on an inner surface of the main body 60A as shown in Fig. 24. The contact portion 60E is shaped to conform to an R-shaped form of the grasping portion 23. Consequently, the main body 60A is placed in surface contact with an outer surface of the grasping portion 23, and thus mounted firmly on the grasping portion 23.

As shown in Fig. 22, hook portions 61a are provided on both flanks of the main body 60A. The hook portions 61 a are designed to be restrained in restraining holes 61b in the mounting belt 61. The mounting belt 61 is made of an elastic material which has a predetermined elasticity. The restraining holes 61b are formed in both sides of the mounting belt 61 to restrain the hook portions 61 a of the main body 60A.

Incidentally, the mounting belt 61 may be made of any material instead of an elastic material as long as the main body 60A can be mounted securely on the grasping portion 23.

The rotary-dial thread fixing unit 60 is fitted over the grasping portion 23 of the endoscope 2 with the contact portion 60E of the main body 60A placed in contact with the surface of the grasping portion 23. After that, the mounting belt 61, with one side mounted on one of the hook portions 61 a of the main body 60A in advance, is wound around the grasping portion 23 as shown in Fig. 22. Then, the other restraining hole 61 b of the mounting belt 61 is fitted over the other hook portion 61 a. Consequently, the rotary-dial thread fixing unit 60 is mounted on the grasping portion 23 as shown in Fig. 21.

On the other hand, as shown in Figs. 21 and 23, the fitting unit 60B is attached to the main body 60A at a predetermined angle to the main body 60A to conform to shape of an entrance portion near the second channel entrance port 22.

Besides, as shown in Figs. 24 to 26, an entrance port 60D is formed in an inner surface between the main body 60A and fitting unit 60B in such a way as to align with the second channel entrance port 22.

As shown in Fig. 23, passage holes 60b are formed in both flanks of the fitting unit 60B to accept the rotating shaft 62a of the rotary dial 62. Also, in that flank on which the rotary dial 62 is arranged, a pair of retaining grooves 60C are provided on opposite sides of the passage hole 60b.

The retaining grooves 60C are designed to be fitted with restraining hooks 63a of the slide lock 63.

The slide lock 63 is formed, for example, by bending a plate member into a U shape as shown in Fig. 23. A fixing nut 64 fixed to the rotating shaft 62a (described later) is placed in an opening portion 63b of the slide lock 63. Also, the restraining hooks 63a are provided on that flank on the opening side of the slide lock 63 which corresponds to the retaining grooves of the fitting unit 60B. When the restraining hooks 63a are fitted in the corresponding retaining grooves 60C, the slide lock 63 is slidably arranged on the fitting unit 60B. Incidentally, longitudinal length of the retaining grooves 60C and length of the restraining hooks 63a are set such that the restraining hooks 63a can move a predetermined amount in the retaining grooves 60C.

Preferably, the slide lock 63 is made of a rigid material. Also, the opening portion 63b and restraining hooks 63a of the slide lock 63 have been dimensioned according to geometries of the fixing nut 64 and the retaining grooves 60C in the fitting unit 60B, respectively.

As shown in Fig. 29, a width dimension of the opening portion 63b of the slide lock 63 is set such that opposite sides of the fixing nut 64 will be placed in the opening portion 63b with some clearance.

Thus, the fixing nut 64 when placed in the opening portion 63b of the slide lock 63 can restrict rotation of the rotating shaft 62a.

As shown in Figs. 27 and 28, the rotary dial 62 is integrally fixed to a proximal end portion 62b of the rotating shaft 62a. The rotating shaft 62a is passed through the passage holes 60b of the fitting unit 60B. The rotating shaft 62a is rotatably arranged in the fitting unit 60B, with the fixing nut 64 and a fixing nut 65 placed on opposite flanks of the fitting unit 60B, where the fixing nut 64 is fixed to a predetermined position on the rotating shaft 62a. The slide lock 63 can slide as indicated by arrow D and the rotary dial 62 can rotate in the illustrated placement location.

Incidentally, the fixing nut 65 is fixed by being screwed onto a threaded portion 62c of the rotating shaft 62a led out of a flank of the fitting unit 60B.

Major components of the rotary-dial thread fixing unit 60 will be described with reference to Fig. 28.

As shown in Fig. 28, the rotating shaft 62a has a groove 66 formed in a location corresponding to the entrance port 60D. The elastic portion 19c provided on the proximal side of the thread-like member 19 is placed in the groove 66.

To place the elastic portion 19c of the thread-like member 19 in the groove 66 of the rotating shaft 62a, the surgeon, for example, inserts the recessed portion 19x of the elastic portion 19c into the groove 66 by gripping an end portion of the thread-like member 19 as shown in Fig. 31. According to the present embodiment, since the groove 66 is configured to be narrower than the recessed portion 19x of the elastic portion 19c, when the recessed portion 19x is pushed into the groove 66, the thread-like member 19 is connected securely to the rotating shaft 62a.

Therefore, during a return operation of the balloon tube member 16, by rotating the rotary dial 62 clockwise as indicated by arrow E in Fig. 28, the surgeon can wind the thread-like member 19 around the rotating shaft 62a.

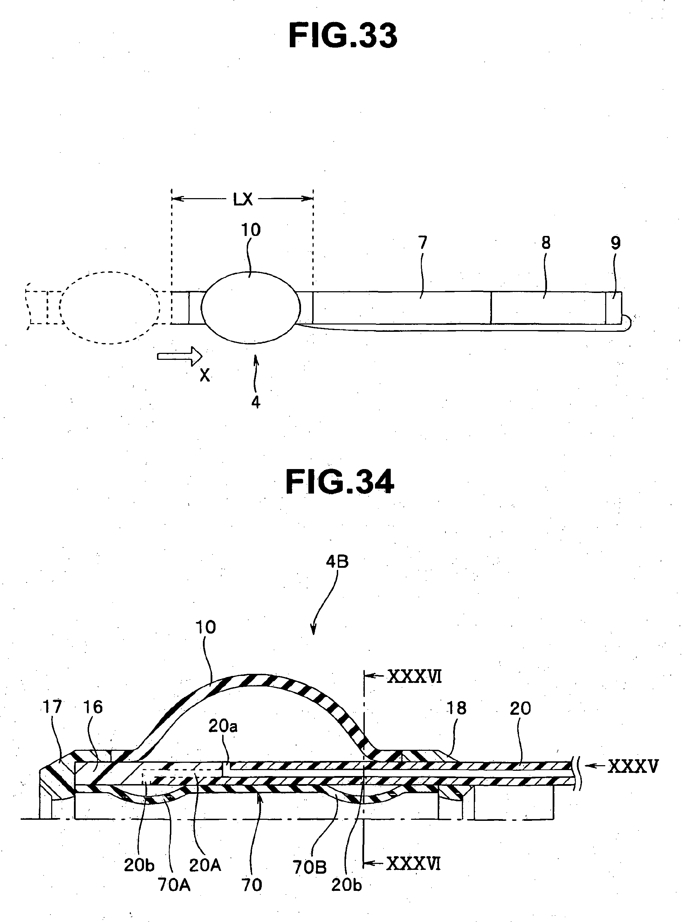

Next, a setup method and characteristic operation of the endoscope insertion aid 4 according to the present embodiment will be described with reference to Figs. 32 and 33.

Fig. 32 is an explanatory diagram showing a maximum stroke through which the balloon tube member can move with respect to the insertion portion and Fig. 33 is an explanatory diagram showing a return of the balloon tube member when the thread-like member is wound by rotating a rotary dial.

According to the present embodiment, the surgeon places the balloon 10 and balloon tube member 16 of the endoscope insertion aid 4 a distance LO away from the rear side of the bending portion 8 as shown in Fig. 32 in contrast to an initial position shown in Fig. 2 and restrains the elastic portion 19c of the thread-like member 19 in the groove 66 of the rotating shaft 62a. Consequently, the balloon tube member 16 is placed away from the insertion portion 3 by the maximum stroke LO.

With the components placed as described above, when the surgeon rotates the rotary dial 62 clockwise, the thread-like member 19 is wound around the rotating shaft 62a. Consequently, the balloon 10 and balloon tube member 16 moves toward the distal side of the insertion portion 3 as indicated by arrow X in Fig. 33.

If travel distance LX of the balloon tube member 16 per rotation of the rotary dial 62 is measured in advance, the travel distance of the balloon tube member 16, for example, in the intestinal tract during a return operation can be recognized in terms of the number of rotations made by the rotary dial 62, making it possible to improve the return operation.

The marker 19b on the thread-like member 19 is intended to let the surgeon know that the balloon tube member 16 is located at the initial position shown in Fig. 2. Thus, when the marker 19b on the thread-like member 19 is wound onto the rotating shaft 62a, the surgeon can restrict the rotation of the rotating shaft 62a by moving the slide lock 63 until the fixing nut 64 is placed in contact with an inner surface of the slide lock 63 and thereby hold the balloon tube member 16 at the initial position shown in Fig. 2.

To perform endoscopy using the endoscope apparatus 1 according to the present embodiment, the distal end portion 9 of the insertion portion 3 is inserted into the intestinal tract with the balloon tube member 16 held at the initial position shown in Fig. 2, in the same way as in the first embodiment.

Then, to advance the insertion portion 3 to a deeper part after hauling in and straightening the intestinal tract such as the sigmoid colon portion 53, for example, as shown in Fig. 15, the surgeon moves the slide lock 63 of the rotary-dial thread fixing unit 60, thereby places the fixing nut 64 out of the opening portion 63b, and thereby derestricts rotation of the rotating shaft 62a and at the same time, derestricts the movement of the balloon tube member 16 with respect to the insertion portion 3.

Then, in the same way as in the first embodiment, the surgeon can perform an insertion operation so as to insert the insertion portion 3 into a deeper part.

Also, for example, to return the balloon tube member 16 to the initial position on the distal side of the insertion portion 3 in order to further insert the insertion portion 3, the surgeon can rotate the rotary dial 62 slowly. That is, the thread-like member 19 is wound around the rotating shaft 62a as the surgeon rotate the rotary dial 62, and consequently the balloon tube member 16 moves toward the distal side of the insertion portion 3 as indicated by a solid line in Fig. 33.

In this case, when it is confirmed that the balloon tube member 16 has returned to a desired position, the surgeon moves the slide lock 63 of the rotary-dial thread fixing unit 60, thereby places the fixing nut 64 in the opening portion 63b again, and thereby restricts the movement of the balloon tube member 16 with respect to the insertion portion 3.

Also, to pull the insertion portion 3 and endoscope insertion aid 4 out of the intestinal tract, the surgeon can do so with the balloon tube member 16 locked using the rotary-dial thread fixing unit 60.

Thus, in addition to the advantage of the first embodiment, the second embodiment provides the advantage of being able to return the balloon 10 and balloon tube member 16 easily and simply in the intestinal tract using the rotary-dial thread fixing unit 60.

Incidentally, according to the present embodiment, multiple thread-like members 19 may be connected to the balloon tube member 16. For example, two thread-like members 19 may be provided in opposing relation to each other on the outer peripheral side of the insertion portion 3, with appropriate channels provided in the insertion portion 3. The use of two thread-like members 19 makes it possible to return the balloon tube member 16 reliably with a smaller tensile force.

An endoscope insertion aid 4B described below can restrict and derestrict the movement of the balloon tube member 16 with respect to the insertion portion 3 without using any of the thread-like member 19, thread fixing unit 30, and rotary-dial thread fixing unit 60 used in the first and/or second embodiments.

### (Third embodiment)

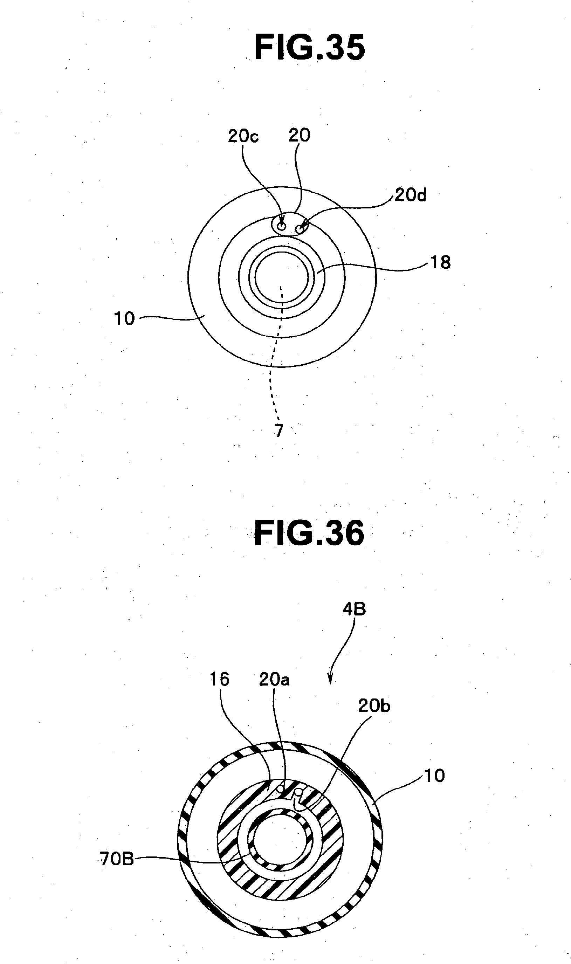

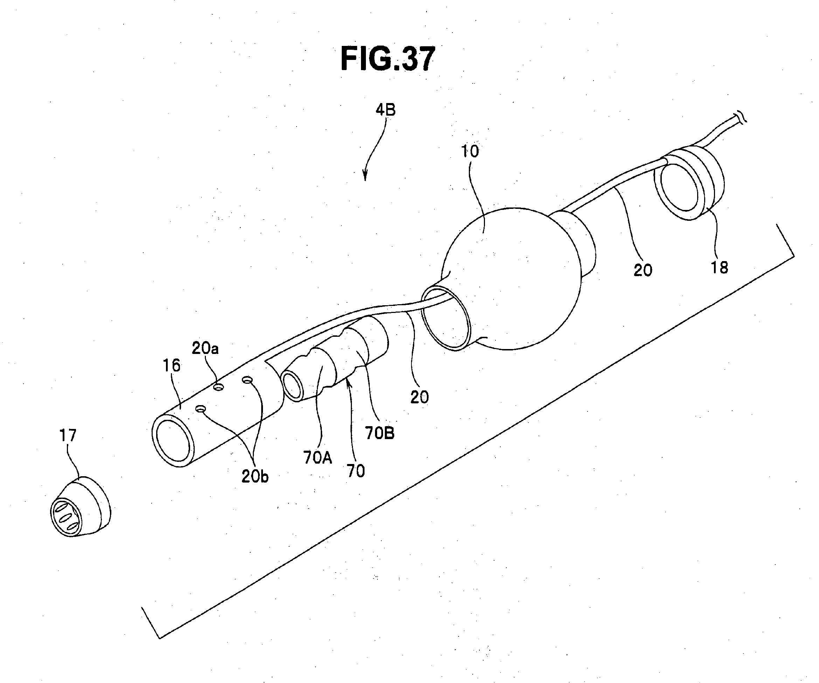

Figs. 34 to 37 concern a third embodiment, where Fig. 34 is a partial cutaway, sectional view illustrating a configuration of an endoscope insertion aid equipped with inner balloons instead of the thread-like member; Fig. 35 is a block diagram as viewed along arrow XXXV in Fig. 34; Fig. 36 is a sectional view taken along line XXXVI - XXXVI in Fig. 34; and Fig. 37 is an exploded perspective view of the endoscope insertion aid in Fig. 34.

In Figs. 34 to 37, the same components as those in the first and/or second embodiments are denoted by the same reference numerals as the corresponding components in the first and/or second embodiments, and description thereof will be omitted. Only differences will be described.

An endoscope apparatus 1 according to the present embodiment is equipped with the endoscope insertion aid 4B shown in Fig. 34. The endoscope insertion aid 4B, which has substantially the same configuration as the endoscope insertion aid 4, includes an inner balloon unit 70 instead of the thread-like member 19 and thread fixing unit 30 used to restrict and derestrict the movement of the balloon tube member 16.

The inner balloon unit 70 includes two inner balloons 70A and 70B which can be inflated and deflated as a fluid is supplied and discharged. Incidentally, the inner balloon unit 70 is not limited to the two inner balloons 70A and 70B, and may have one or more than two balloons.

The two inner balloons 70A and 70B are provided on an inner peripheral side of the balloon tube member 16 as shown in Figs. 34 and 36.

As shown in Fig. 35, the fluid tube 20 provided for the balloon tube member 16 includes an inner-supply conduit 20d used to supply the fluid to the inner balloons 70A and 70B as well as an outer-supply conduit 20c used to supply the fluid to the balloon 10. The inner-supply conduit 20d has openings 20b which are communicated with hollow portions in the inner balloons 70A and 70B, respectively, as shown in Figs. 34 and 36, to supply and discharge the fluid to/from the hollow portions.

Since the fluid tube 20 according to the present embodiment has two different conduits--the inner-supply conduit 20d and outer-supply conduit 20c, two balloon control pumps 33 are provided in a rear end portion of the fluid tube 20, the one being connected to the outer-supply conduit 20c and the other being connected to the inner-supply conduit 20d. This makes it possible to control inflation/deflation of the outer balloon 10 and inflation/deflation of the inner balloons 70A and 70B separately.

An assembly method of the endoscope insertion aid 4B is substantially the same as the first embodiment. The inner balloon unit 70 is fixed to the inner peripheral side of the balloon tube member 16 with an adhesive or the like as shown in Fig. 37. Incidentally, the endoscope insertion aid 4B does not need to connect the thread-like member 19.

Regarding operation of the present embodiment, instead of restricting and derestricting the movement of the balloon tube member 16 using the thread-like member 19 and thread fixing unit 30 as in the case of the first embodiment, the endoscope apparatus 1 according to the present embodiment restricts and derestricts the movement of the balloon tube member 16 with respect to the insertion portion 3 by inflating and deflating the inner balloons 70A and 70B of the inner balloon unit 70 via fluid supply and discharge thereto/therefrom.

The rest of the operation is the same as the first embodiment.

Thus, the third embodiment can restrict and derestrict the movement of the balloon tube member 16 with respect to the insertion portion 3 using the inner balloon unit 70 instead of the thread-like member 19 and thread fixing unit 30 according to the first embodiment, and thereby provide the same advantage as the first embodiment.

## Claims

1. An endoscope insertion aid (4) comprising:
a tubular member (16) which allows an insertion portion (3) of an endoscope (2) to be passed through and can move forward and backward relatively along an insertion axis of the passed insertion portion (3);
a holding unit (10) which, being provided on an outer peripheral side of the tubular member (16), can be inflated and deflated with supply and discharge of a fluid;
a thread-like member (19) passed through a channel (15) in the insertion portion (3), from an opening portion (15a) on a distal end side of the endoscope (2) to an opening (22) on a proximal end side of the endoscope (2), the thread-like member (19) having one end connected to the tubular member (16) and the other end extended from the opening (22);
a thread-like member fixing unit (30) which restrains the thread-like member (19) extending from the channel opening (22) arranged on the proximal end side of the endoscope (2) and thereby restricts movement of the tubular member (16) toward the proximal end side with respect to the insertion portion (3) along the insertion axis; **characterized by**
a restraining member (20) which supplies and discharges the fluid into/from the holding unit (10), being a tube/conduit member being connected at one end to thetubular member (16), and extending at the other end to the proximal end side along an outer peripheral side of the insertion portion (3); and
a restraining member fixing unit (31) which holds the tubular member (16) at a desired position in an intestinal tract by fixing the other end of the restraining member (20).

2. The endoscope insertion aid (4) according to claim 1, wherein the thread-like member (19) and the restraining member (20) have flexibility.

3. The endoscope insertion aid (4) according to claim 1 or 2, wherein the thread-like member fixing unit (30) fixes the tubular member (16) at a desired position relative to the insertion portion (3) along an insertion direction by restraining the thread-like member (19) and allows the tubular member (16) to move forward and backward relative to the insertion portion (3) along the insertion direction by releasing the restraint on the thread-like member (19).

4. The endoscope insertion aid (4) according to any one of claims 1 to 3, wherein the restraining member fixing unit (31) holds the tubular member (16) at the desired position in the intestinal tract by fixing the restraining member (20) and allows the tubular member (16) to move forward and backward in the intestinal tract by releasing the restraining member (20).

5. An endoscope apparatus comprising:
an endoscope (2) equipped with an insertion portion (3) which, having an elongated shape, can be inserted into an intestinal tract, wherein a channel (15) is arranged inside of the insertion portion (3) along a longitudinal direction of the insertion portion (3);
an endoscope insertion aid (4) according to claim 1; and
fluid supply/discharge means (33) which, being connected to the restraining member (20), supplies and discharges the fluid into/from the holding unit (10) via the restraining member (20) and thereby inflates and deflates the holding unit (10).

6. The endoscope apparatus according to claim 5, wherein the opening portion (15a) communicated with the channel (15) in the insertion portion (3) is an opening provided in outer peripheral surface on a proximal side of a bending portion of the insertion portion (3) or an opening which opens to a distal end face of a distal end portion of the insertion portion (3).

## Patentansprüche

1. Endoskop-Einführhilfe (4), mit:
einem schlauchförmigen Element (16), durch das ein Einführabschnitt (3) eines Endoskops (2) durchgeführt und vorwärts und rückwärts relativ entlang einer Einführachse des durchgeführten Abschnitts (3) bewegt werden kann;
einer Halteeinheit (10), die da sie an einer Außenumfangsseite des schlauchförmigen Elements (16) vorgesehen ist, durch die Zufuhr und das Ausleiten eines Fluids gefüllt und entleert werden kann;
einem fadenartigen Element (19), das durch einen Kanal (15) im Einführabschnitt (3) von einem Öffnungsabschnitt (15a) an der Seite des distalen Endes des Endoskops (2) zur einer Öffnung (22) an der Seite des proximalen Endes des Endoskops (2) geführt wird, wobei das fadenartige Element (19) ein Ende hat, das mit dem schlauchförmigen Element (16) verbunden ist und das andere Ende sich von der Öffnung (22) aus erstreckt;
einer Fixiereinheit (30) für das fadenartige Element, die das fadenartige Element (19), das sich aus der Kanalöffnung (22) an der Seite des proximalen Endes des Endoskops (2) erstreckt, sichert und dadurch die Bewegung des schlauchförmigen Elements (16) zur Seite des proximalen Endes bezüglich des Einführabschnitts (3) entlang der Einführachse einschränkt; **gekennzeichnet durch**
einen Fluidschlauch (20), der der Halteeinheit (10) das Fluid zuführt und aus dieser ausleitet, bei dem es sich um ein Schlauch-/Leitungselement handelt, das an einem Ende mit dem schlauchförmigen Element (16) verbunden ist und dessen anderes Ende sich zur Seite des proximalen Endes entlang der Außenumfangsseite des Einführabschnitts (3) erstreckt; und
eine Fixiereinheit (31) für den Fluidschlauch, die das schlauchförmige Element (16) an einer gewünschten Position im Magen-Darm-Trakt **durch** Fixieren des anderen Endes des Fluidschlauchs (20) hält.

2. Endoskop-Einführhilfe (4) nach Anspruch 1, wobei das fadenartige Element (19) und der Fluidschlauch (20) flexibel sind.

3. Endoskop-Einführhilfe (4) nach Anspruch 1 oder 2, wobei die Fixiereinheit (30) für das fadenartige Element das schlauchförmige Element (16) an einer gewünschten Position relativ zum Einführabschnitt (3) entlang der Einführrichtung durch Sichern des fadenartigen Elements (19) fixiert und es dem schlauchförmigen Element (16) ermöglicht, sich relativ zum Einführabschnitt (3) entlang der Einführrichtung vorwärts und rückwärts zu bewegen, indem die Sicherung des fadenartigen Elements (19) freigegeben wird.

4. Endoskop-Einführhilfe (4) nach einem der Ansprüche 1 bis 3, wobei die Fixiereinheit (31) für den Fluidschlauch das schlauchförmige Element (16) an der gewünschten Position im Magen-Darm-Trakt durch Fixieren des Fluidschlauchs (20) hält und es dem schlauchförmigen Element (16) ermöglicht, sich im Magen-Darm-Trakt vorwärts und rückwärts zu bewegen, indem die Sicherung des Fluidschlauchs (20) freigegeben wird.

5. Endoskopvorrichtung, mit:
einem Endoskop (2), das mit einem Einführabschnitt (3) ausgerüstet ist, der da er eine langgezogene Form hat, in einen Magen-Darm-Trakt eingeführt werden kann, wobei ein Kanal (15) im Innern des Einführabschnitts (3) entlang der Längsrichtung des Einführabschnitts (3) angeordnet ist;
einer Endoskop-Einführhilfe (4) nach Anspruch 1; und
einem Fluid-Zufuhr-/Ausleitmittel (33), das da es mit dem Fluidschlauch (20) verbunden ist, das Fluid über den Fluidschlauch (20) in die Halteeinheit (10) liefert und aus dieser ausleitet und dadurch die Halteeinheit (10) füllt und entleert.

6. Endoskopvorrichtung nach Anspruch 5, wobei der Öffnungsabschnitt (15a), der mit dem Kanal (15) im Einführabschnitt (3) kommuniziert, eine Öffnung in der Außenumfangsfläche an einer proximalen Seite eines Biegeabschnitts des Einführabschnitts (3) ist, oder eine Öffnung, die in eine distale Stirnfläche eines distalen Endabschnitts des Einführabschnitts (3) mündet.

## Revendications

1. Dispositif (4) d'aide à l'insertion d'un endoscope comprenant :
un élément tubulaire (16) qui permet qu'une partie d'insertion (3) d'un endoscope (2) soit passée à travers et peut se déplacer vers l'avant et vers l'arrière de manière relative le long d'un axe d'insertion de la partie d'insertion (3) passée ;
une unité de maintien (10) qui, étant prévue sur un côté périphérique externe de l'élément tubulaire (16), peut être gonflée et dégonflée avec l'alimentation et l'évacuation d'un fluide ;
un élément en forme de fil (19) passé à travers un canal (15) dans la partie d'insertion (3), d'une partie d'ouverture (15a) sur un côté d'extrémité distale de l'endoscope (2) à une ouverture (22) sur un côté d'extrémité proximale de l'endoscope (2), l'élément en forme de fil (19) comportant une extrémité connectée à l'élément tubulaire (16) et l'autre extrémité s'étendant depuis l'ouverture (22) ;
une unité de fixation (30) d'élément en forme de fil qui retient l'élément en forme de fil (19) s'étendant depuis l'ouverture de canal (22) agencée sur le côté d'extrémité proximale de l'endoscope (2) et limite de ce fait le déplacement de l'élément tubulaire (16) vers le côté d'extrémité proximale par rapport à la partie d'insertion (3) le long de l'axe d'insertion ;
**caractérisé par**
un élément de retenue (20) qui délivre et évacue le fluide dans/depuis l'unité de maintien (10), étant un élément de tube/conduit étant connecté au niveau d'une extrémité à l'élément tubulaire (16), et s'étendant au niveau de l'autre extrémité vers le côté d'extrémité proximale le long d'un côté périphérique externe de la partie d'insertion (3) ; et
une unité de fixation (31) d'élément de retenue qui maintient l'élément tubulaire (16) au niveau d'une position souhaitée dans un tract intestinal en fixant l'autre extrémité de l'élément de retenue (20).

2. Dispositif (4) d'aide à l'insertion d'un endoscope selon la revendication 1, dans lequel l'élément en forme de fil (19) et l'élément de retenue (20) sont flexibles.

3. Dispositif (4) d'aide à l'insertion d'un endoscope selon la revendication 1 ou 2, dans lequel l'unité de fixation (30) d'élément en forme de fil fixe l'élément tubulaire (16) au niveau d'une position souhaitée par rapport à la partie d'insertion (3) le long d'une direction d'insertion en retenant l'élément en forme de fil (19) et permet à l'élément tubulaire (16) de se déplacer vers l'avant et vers l'arrière par rapport à la partie d'insertion (3) le long de la direction d'insertion en libérant la retenue sur l'élément en forme de fil (19).

4. Dispositif (4) d'aide à l'insertion d'un endoscope selon l'une quelconque des revendications 1 à 3, dans lequel l'unité de fixation (31) d'élément de retenue maintient l'élément tubulaire (16) au niveau de la position souhaitée dans le tract intestinal en fixant l'élément de retenue (20) et permet à l'élément tubulaire (16) de se déplacer vers l'avant et vers l'arrière dans le tract intestinal en libérant l'élément de retenue (20).

5. Appareil d'endoscope comprenant :
un endoscope (2) muni d'une partie d'insertion (3) qui, présentant une forme allongée, peut être insérée dans un tract intestinal, dans lequel un canal (15) est agencé à l'intérieur de la partie d'insertion (3) le long d'une direction longitudinale de la partie d'insertion (3) ;
un dispositif (4) d'aide à l'insertion d'un endoscope selon la revendication 1 ; et
un moyen d'alimentation/évacuation de fluide (33) qui, étant connecté à l'élément de retenue (20), délivre et évacue le fluide dans/depuis l'unité de maintien (10) via l'élément de retenue (20) et de ce fait gonfle et dégonfle l'unité de maintien (10).

6. Appareil d'endoscope selon la revendication 5, dans lequel la partie d'ouverture (15a) en communication avec le canal (15) dans la partie d'insertion (3) est une ouverture prévue dans une surface périphérique externe sur un côté proximal d'une partie de courbure de la partie d'insertion (3) ou une ouverture qui s'ouvre sur une face d'extrémité distale d'une partie d'extrémité distale de la partie d'insertion (3).
